# EUROPEAN PATENT SPECIFICATION

(11) **EP 0 602 899 B1**
(45) Date of publication and mention of the grant of the patent: **20.10.2004**
(21) Application number: 93309920.2
(22) Date of filing: 09.12.1993
(51) Int. Cl.: C12N 15/62, C07K 14/00, C12N 15/66, C12N 9/22

(54) **Modified proteins containing controllable intervening sequences and methods of their production**
Kontrollierbare Zwischensequenzen enthaltende modifizierte Proteine und Verfahren zur deren Herstellung
Protéines modifiées contenant des séquences interjacentes contrôlables et méthode pour leurs productions

(30) Priority: 09.12.1992 US 4139; 03.11.1993 US 146885
(43) Date of publication of application: 22.06.1994
(60) Divisional application: 04075210.7
(73) Proprietor: NEW ENGLAND BIOLABS, INC., Beverly Massachusetts 01915 (US)
(72) Inventor: Comb, Donald G., Beverly, Massachusetts 01915 (US); Perler, Francine B., Brookline, Massachusetts 02146 (US); Jack, William E., Wenham, Massachusetts 01984 (US); Hodges, Robert A., Manassas, Virginia 22110 (US); Xu, Ming-Qun, Rockport House, Beverly, Massachusetts 01915 (US); Noren, Christopher J., Rockport, Massachusetts 01966 (US)
(74) Representative: Froud, Clive

(56) References cited:
- EP-A- 0 475 185
- WO-A-88/04692
- CELL., vol.71, October 1992, CAMBRIDGE, NA US pages 201 - 210 E.O.DAVIS ET AL. 'PROTEIN SPLICING...'
- PROCEEDINGS OF THE NATIONAL ACADEMY OF SCIENCES OF USA., vol.89, June 1992, WASHINGTON US pages 5577 - 5581 F.B.KELLER ET AL. 'INTERVENING SEQUENCES...'
- SCIENCE., vol.250, 1990, LANCASTER, PA US pages 651 - 657 P.M.KANE ET AL. 'PROTEIN SPLICING...'
- FEBS LETTERS, vol.263, no.1, 1990, AMSTERDAM NL pages 23 - 26 S.J.WEARNE 'FACTOR XA CLEAVAGE...'
- JOURNAL OF BIOLOGICAL CHEMISTRY., vol.264, no.7, 1989, BALTIMORE US pages 3666 - 3669 D.R.COREY ET AL. 'INTRODUCTION OF...'
- NUCLEIC ACIDS RESEARCH, vol.20, no.23, 11 December 1992, ARLINGTON, VIRGINIA US pages 6153 - 6157 R.A.HODGES ET AL. 'PROTEIN SPLICING...'

## Description

### CROSS-REFERENCE TO RELATED APPLICATION

This Application is a Continuation-In-Part Application of co-pending Application Serial No. 08/004,139, filed December 9, 1992.

### BACKGROUND OF THE INVENTION

The present invention is directed to modified proteins and methods of producing the same. More specifically, the modified protein of the present invention comprises a target protein and a controllable intervening protein sequence (CIVPS), the CIVPS being capable of excision or cleavage under predetermined conditions.

Production of mature proteins involves the flow of information from DNA to RNA to protein. Precise excision of DNA and RNA elements which interrupt that information has been previously described [M. Belfort, *Annu*. *Rev*. *Genet*. 24:363 (1990); T.R. Cech, *Annu*. *Rev*. *Biochem.* 59:543 (1990); Hunter et al., *Genes Dev.* 3:2101 (1989)]. More.recently, evidence for the precise excision of intervening protein sequences has also been described for the *TFPI* allele from *Saccharomyces cerevisiae* [Hirata et al., *J*. *Biol. Chem*. 265:6726 (1990); Kane et al., *Science* 250:651 (1990)] and the *rec A* gene from *Mycobacterium tuberculosis* [Davis et al., *J. Bact.* 173:5653 (1991); Davis et al., *Cell* 71:1 (1992)]. Each contains internal in-frame peptide segments which must be removed to produce the mature protein. Expression of Tfp1 and Rec A each results in two peptides: one representing the intervening protein sequence (IVPS) and the other the ligated product of the external protein sequences (EPS). This post-translational processing event has been termed "protein splicing". Similarly, the Vent DNA polymerase gene from the hyperthermophilic archaea *Thermococcus litoralis* contains two in-frame IVPSs [Perler, et al., *PNAS* 89:5577 (1992)].

A major impediment to the development of methods of using IVPSs or protein splicing in other than research applications has been the inability to control the activity of the IVPS and thus the splicing event.

Thus, it would be desirable to have a method which provides a ready means to modify a target protein using an IVPS, particularly where the activity of the IVPS is controllable. It would also be desirable to have a method which can specifically modify target proteins such that their activity is substantially inactivated. It would be desirable to have a method which can be used to restore the activity of an inactivated modified protein.

### SUMMARY OF THE INVENTION

The present invention relates to modified proteins comprising an IVPS and a target protein, the IVPS being capable of excision by protein splicing, or cleavage in the absence of splicing, under predetermined conditions. Such predetermined conditions depend on the IVPS used and can include, for example, increase in temperature, changes in pH conditions, exposure to light, dephosphorylation, or deglycosylation of amino acid residues. The IVPS may be joined with the target protein either by inserting the IVPS into the target protein or fusing the IVPS with the target protein at either the amino or carboxy terminal end of the target protein. These IVPS, referred to as controllable intervening protein sequences (CIVPS), are therefore useful in controlling the splicing or cleavage reaction. The present invention further relates to methods for producing, selecting and testing CIVPSs.

In one preferred embodiment, a DNA sequence encoding a CIVPS is inserted into, or joined with, a DNA sequence encoding a target protein such that both coding sequences form a continuous open reading frame. Thereafter, expression of this fusion DNA is utilized to produce the modified target protein. In another embodiment, the modified protein so produced is subjected to predetermined conditions under which the CIVPS will be excised or cleaved. In certain embodiments, the CIVPS is inserted into a region of the target protein which renders the target protein substantially inactive and excision of the CIVPS restores the activity of the target protein.

Preferred CIVPSs include CIVPS1 and 2 obtainable from *T*. *litoralis* (also sometimes referred to as Vent IVPS 1 and 2 or IVS1 and 2) and CIVPS 3 obtainable from *Pyrococcus* sp.(also sometimes referred to as Deep Vent IVPS1 or IVS1). These CIVPSs are capable of excision, i.e., removal via protein splicing, from modified proteins upon an increase in temperature.

In accordance with the present invention, it has also been found that certain CIVPS amino acid residues and at least the first downstream amino acid residue modulate the splicing reaction and that modification of these residues decreases or stops the splicing reaction. These residues have been shown to be conserved in other IVPSs. Modification of such residues can be used to convert a IVPS to a CIVPS.

In accordance with the present invention, it has been found that in certain situations, the complete splicing reaction is not necessary or desirable. In such situations, the CIVPS can be modified to allow cleavage in the absence of splicing, thus allowing for controlled separation or cleavage of the CIVPS from the target protein.

The potential uses for the modified proteins and CIVPSs of the present invention are manifold. These include, for example, control of a target protein's enzymatic activity, purification of modified proteins using antibodies specific to the CIVPS by affinity chromatography and production of proteins that are toxic to host cells.

The CIVPSs of the present invention may further be used in a method of protein purification in which a modified protein comprising a target protein fused to a CIVPS is produced. If desired, a three-part fusion can be produced in which the CIVPS is between the target protein and a protein having affinity for a substrate (binding protein), e.g., MBP. The modified protein is then contacted with a substrate to which the CIVPS or binding protein has specific affinity, e.g., using affinity chromatography. The highly purified target protein can be liberated from the column by subjecting the CIVPS to predetermined conditions under which cleavage, for example, between the CIVPS and the target protein is initiated. Alternatively, the fusion protein can be purified as above and then the target protein released from the fusion by subjecting the CIVPS to predetermined conditions.

### BRIEF DESCRIPTION OF THE DRAWINGS

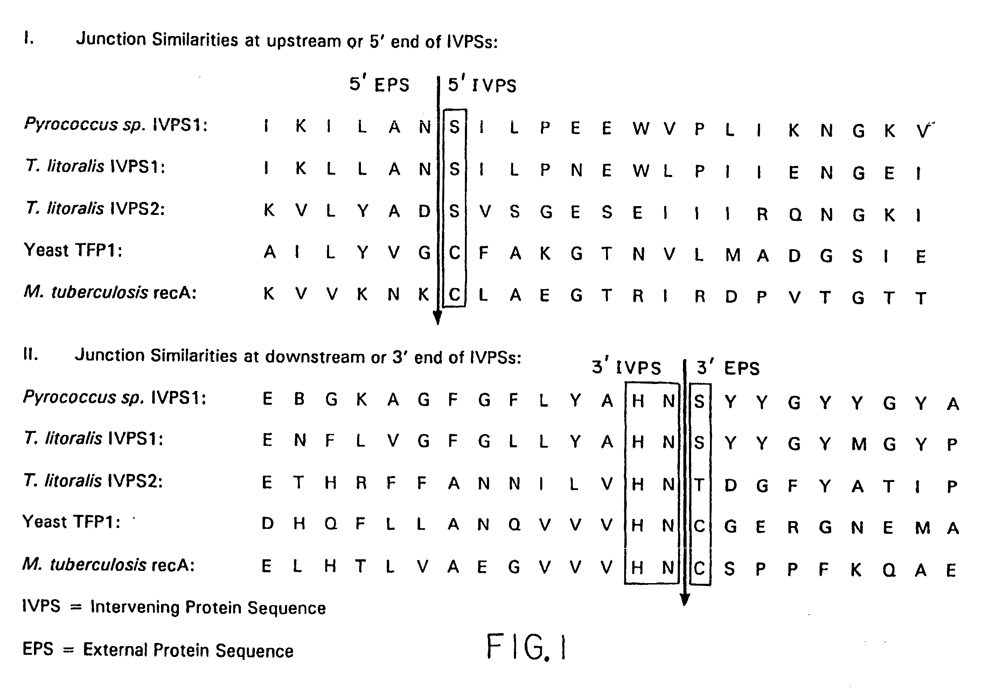

Figure 1 depicts the amino acid sequence (SEQ ID NO:30, SEQ ID NO:31, SEQ ID NO:32, SEQ ID NO:33, SEQ ID NO:34, SEQ ID NO:36, SEQ ID NO:37, SEQ ID NO:38 and SEQ ID NO:39) of proposed protein splice junctions. Amino-terminal (top) and carboxy-terminal (bottom) splice junctions are shown with splice sites indicated by arrows and conserved or similar amino acids boxed.

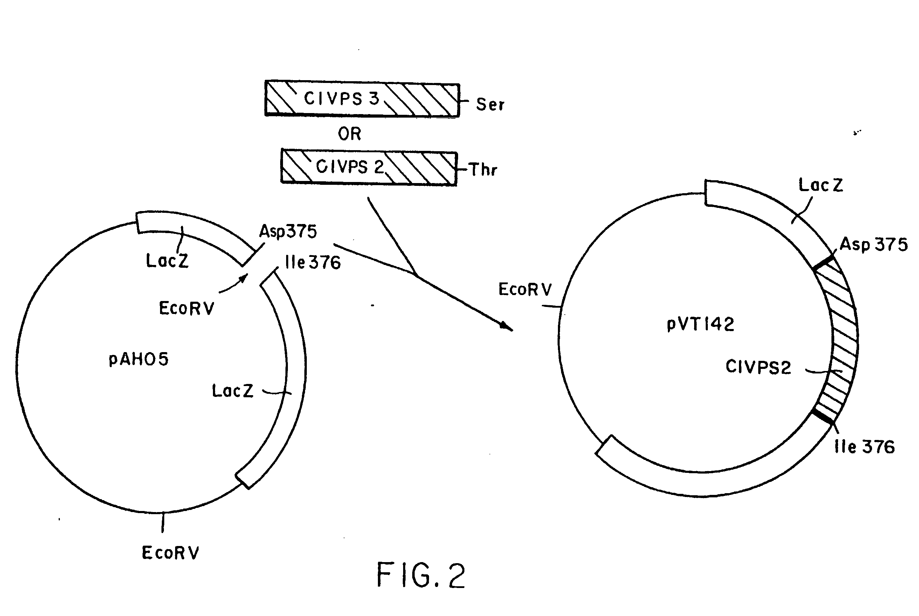

Figure 2 illustrates insertion of IVPS into the *EcoRV* site of the β-galactasidase gene. PCR products of either Deep Vent IVPS1 (CIVPS3) or Vent IVPS2 (CIVPS2) are ligated to *Eco*RV digested pAHO5 between the Asp and lle residues of β-galactosidase to produce a modified β-galactosidase product.

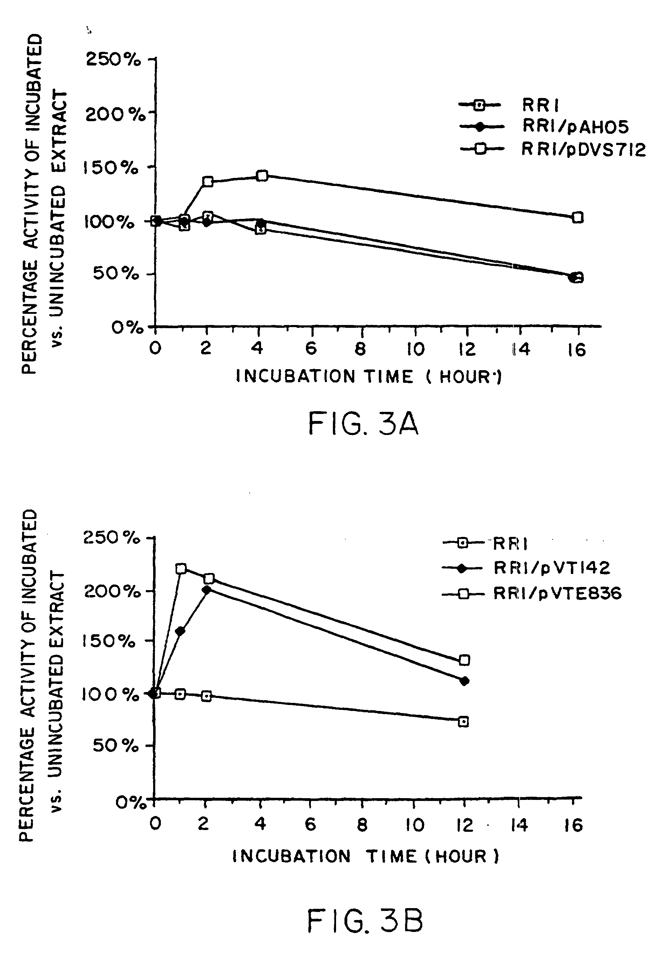

Figure 3 are graphs showing that splicing of modified β-galactosidase yields active β-galactosidase. Incubation of crude extracts from hosts expressing the indicated IVPS-β-galactosidase fusion proteins at 42°C yields an increase in enzyme activity with time, whereas incubation at 42°C with the host alone (RR1) or an unmodified β-galactosidase construct (pAHO5) shows no increase in enzyme activity.

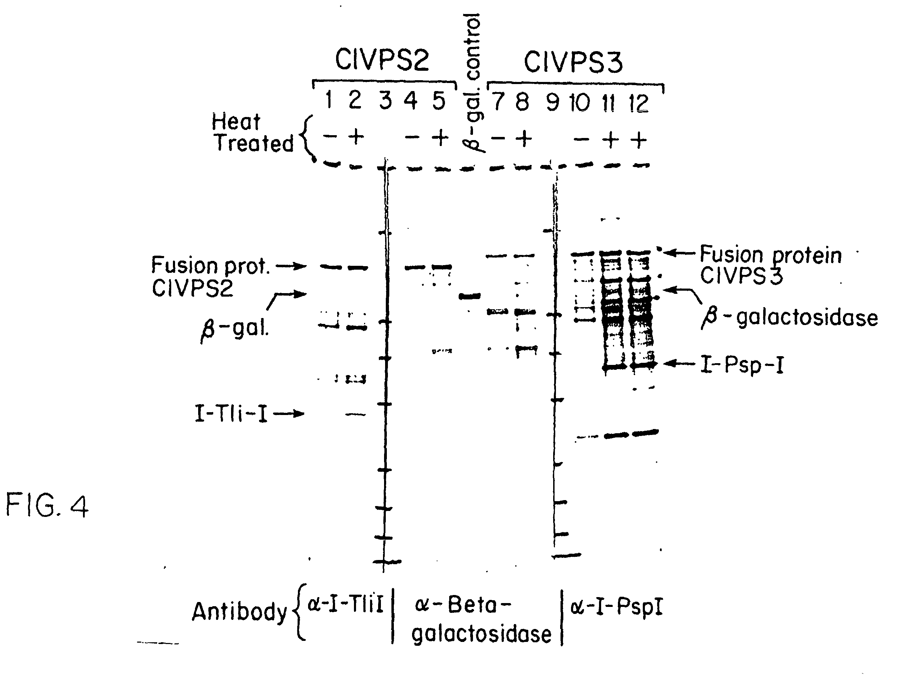

Figure 4 is a western blot showing the results of temperature controlled protein splicing experiments. CIVPS2 and CIVPS3 were cloned into the *Eco*RV site of β-galactosidase. Western blot examination of cell extracts with sera directed against β-galactosidase or the CIVPS protein (I*-Tli*l and I-*Psp*I, respectively) detects modified β-galactosidase fusion protein (Lanes 1,4,7,10). Treatment of extracts at 42°C (Lanes 2,5,8,11) or 50°C (Lane 12) for 6 hours results in splicing and the production of free CIVPS proteins and unmodified β-galactosidase (except for retained serine or threonine residue, see text example 2 & 3). Unmodified β-galactosidase from pAHO5 is in lane 6. Lanes 3 & 9 contain size markers.

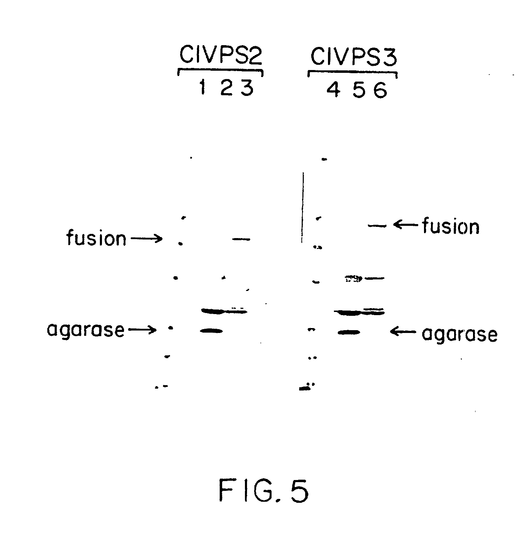

Figure 5 shows by western blot examination of cell extracts with sera directed against β-agarase, the detection of modified β-agarase fusion protein. Lanes 1 & 4: size markers; Lanes 2 & 5: β-agarase standard; lane 3: CIVPS2-β-agarase fusion; lane 6: CIVPS3-β-agarase fusion.

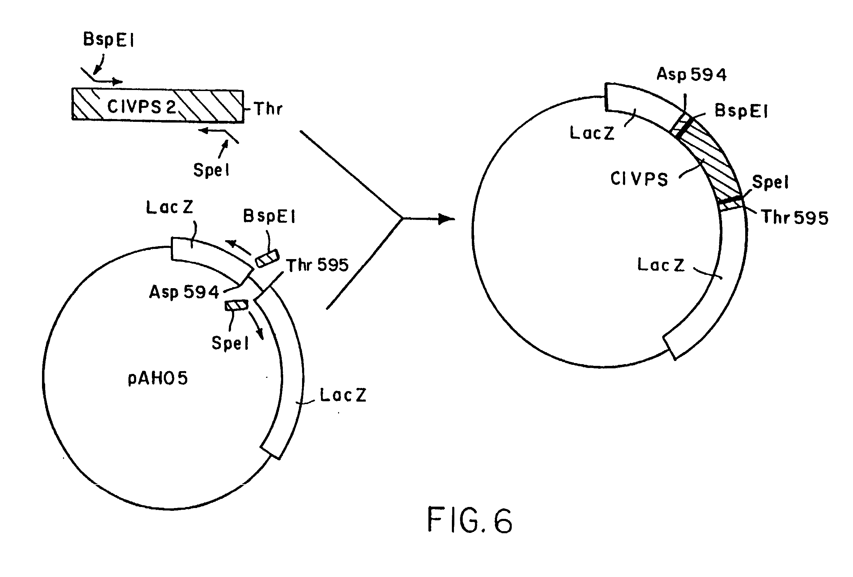

Figure 6 illustrates insertion of IVPS2 (CIVPS2) into the β-galactasidase gene by creation of new restriction sites (*Bsp*EI and *Spe*l) within the IVPS by silent mutations.

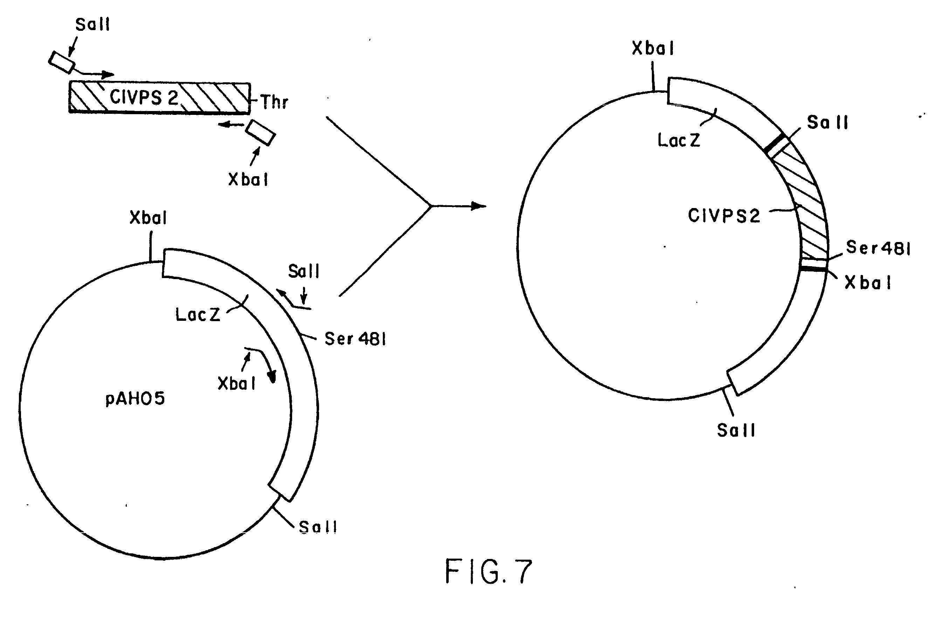

Figure 7 illustrates insertion of either Deep Vent IVPS1 (CIVPS3) or Vent IVPS2 (CIVPS2) into the β-galactasidase gene by creation of new restriction sites (*Xba*I and *Sal*I) by silent mutations within the target gene.

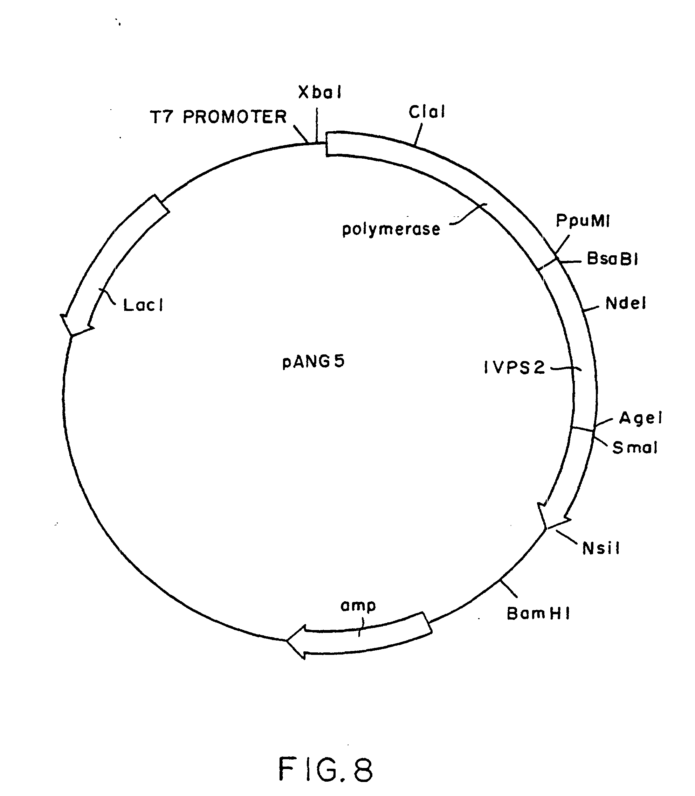

Figure 8 is a plasmid map of pANG5.

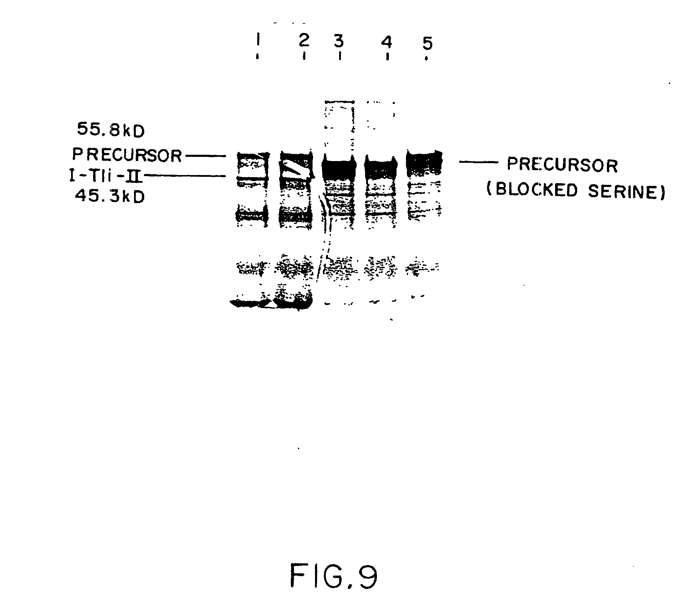

Figure 9 is an autoradiogram of SDS-PAGE showing suppressor tRNA-mediated incorporation of a chemically blocked serine at the upstream junction of CIVPS2.

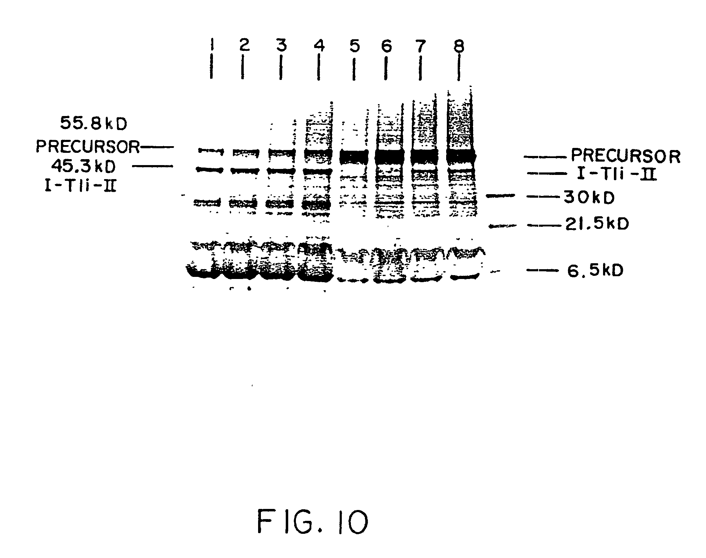

Figure 10 is an autoradiogram of SDS-PAGE showing the splicing reaction of CIVPS2 initiated by visible light irradiation of a chemically blocked precursor protein.

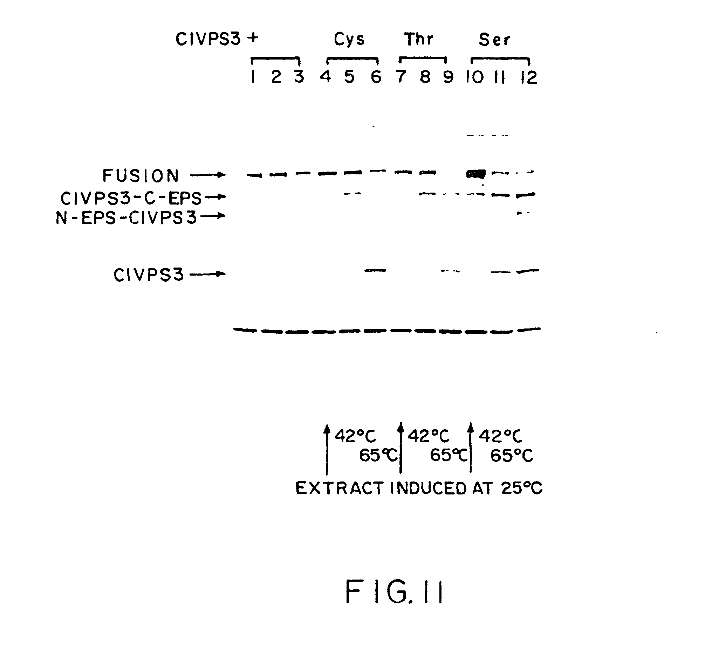

Figure 11 is a gel showing temperature controlled protein splicing and cleavage. Deep Vent IVPS1 (CIVPS3) cassettes were cloned into the *Eco*RV site of β-galactosidase. Western blot analysis was used to examine cell extracts of pDV7 (CVPS3 cassette, lanes 1-3), pDVC302 (CIVPS3/Cys cassette, lanes 4-6), pDVT321 (CIVPS3/Thr cassette, lanes 7-9) and pDVS712 (CIVPS3/Ser cassette, lanes 10-12). Antibody directed against the CIVPS3 protein (I-*Psp*I) (NEB) detects fusion proteins and cleavage products including free CIVPS3, N-EPS-CIVPS3 and CIVPS3-C-EPS (from cleavage at one of the splice junctions). The untreated extracts were in lanes 1, 4, 7, and 10. Treatment of extracts at 42°C (lanes 2, 5, 8, and 11) or 65°C (lanes 3, 6, 9, and 12) for 2 hours results in increased splicing and/or cleavage activity at different efficiency.

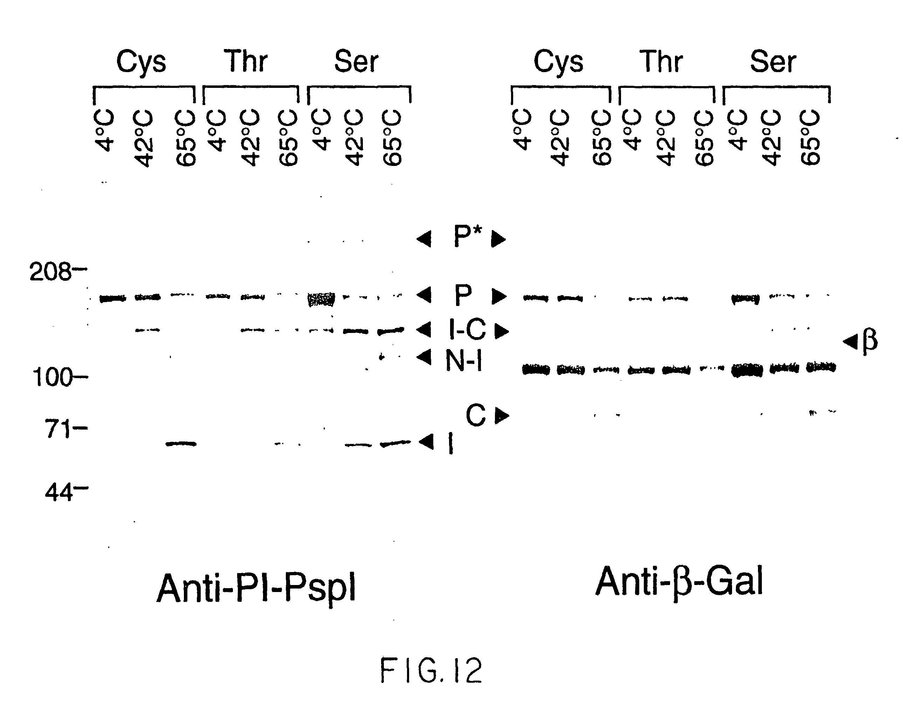

Figure 12 is a Western blot showing temperature controlled protein splicing and cleavage. Western blot analysis using antibody directed against I-*Psp*I and β-galactosidase (C-EPS domain) (Promega) were used to examine fusion constructs pDVC302 (lanes 1-3), pDVT321 (lanes 4-6) and pDVS712 (lanes 7-9). Treatment of extracts at 42°C (lanes 2, 5, and 8) or 65°C (lanes 3, 6, and 9) for 2 hours results in splicing (in pDVS712) or cleavage. Protein splicing in pDVS712 extract produced free CIVPS3 protein, I-*Psp*I and unmodified β-galactosidase (except for retained serine). Lane 1 contains size markers.

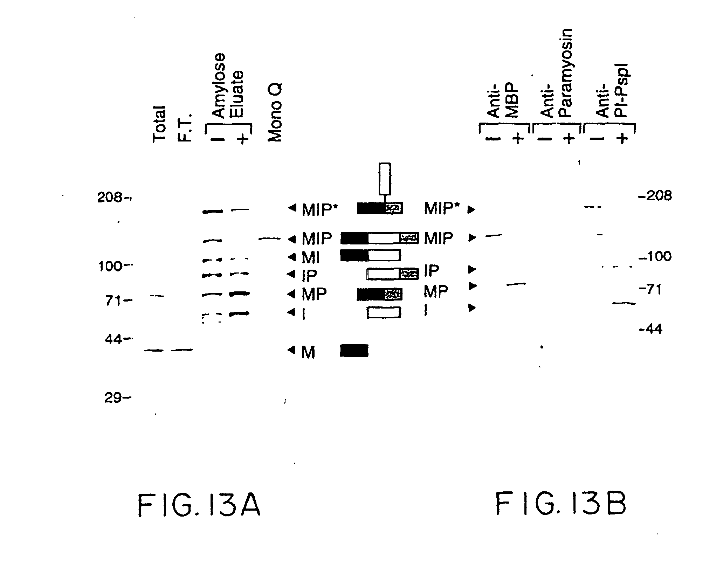

Figure 13 shows the purification of MIP precursor on amylose and MonoQ columns examined by Coomassie blue staining and immunoblot. The diagram between Parts A and B represents the proposed structure of each band, including the branched molecule MIP*. The black boxes represent the MBP domain, the white boxes the IVPS doman and the gray boxes the paramyosin ΔSaI domain. The pluses (+) indicate that the sample was heat treated at 37°C for 120 min., minuses (-) indicate that the sample was not heat treated. Part A: Coomassie blue stained gel. Total, crude supernatants from MIP cultures. F.T., amylose resin flow through. Amylose eluate (-), amylose resin purified MIP preparations. Amylose eluate (+), the amylose eluate in lane 4 was treated at 37°C for 120 min. to induce splicing. MonoQ, MonoQ purified sample. After chromatography on MonoQ, recovery of MBP-CIVPS3 (M!) was variable and generally low. Symbols are as follows: MIP*, 180 kDa apparent molecular mass branched molecule; MIP, 132 kDa precursor; single splice junction cleavage products (MI, MBP-CIVPS3; IP, CIVPS3-paramyosin ΔSal; M, MBP); and spliced products (MP, MBP-paramyosin ΔSal and I, CIVPS3 = PI-*Psp*I). Part B: Immunoblots. The MonoQ sample from Part A was heat treated as above and electrophoresed in triplicate. MIP-related proteins were identified by immune reactivity with anti-MBP sera, anti-paramyosin sera and anti-PI-*Psp*l (anti-CIVPS3) sera.

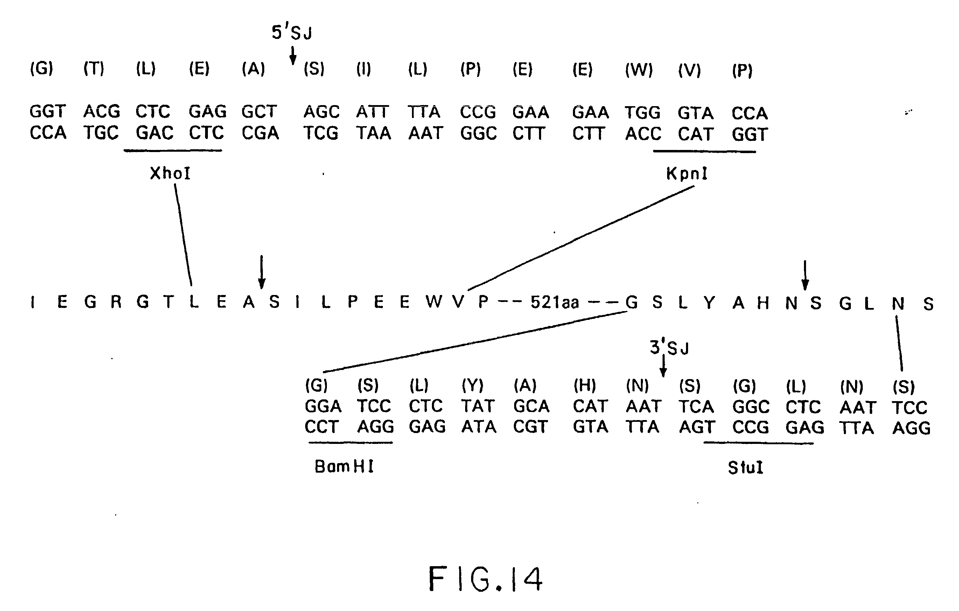

Figure 14 illustrates the replaceable splice junction cassettes in MIP21 fusion. pMIP21 contains two unique restriction sites flanking each splice junction. Splice junctions are indicated by arrows. Amino acid residues around the splice junctions are shown. Splice junctions can be changed by replacing either the amino terminal *Xho*I-*Kpn*I cassette or the carboxyl terminal *Bam*HI-*Stu*I cassette with another DNA cassette.

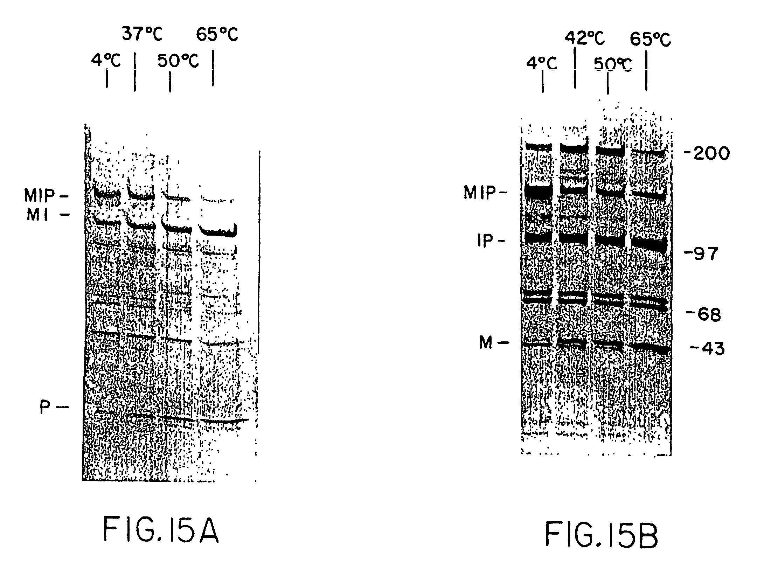

Figure 15 is a gel showing thermal inducible cleavage at a single splice junction from modified MIP fusions. Fusion proteins were purified using amylose resin columns.
Figure 15A shows cleavage at the C-terminal splice junction from MIP23 fusion. Purified fusion protein samples were incubated at 4°C, 37°C, 50°C or 65°C for 1 hour. Products were analyzed by a 4/20% SDS-PAGE followed by Coomassie blue staining. Cleavage of the C-terminal splice junction of the MIP23 fusion protein (MIP) yielded MBP-CIVPS (MI) and paramyosin ΔSal (P).
Figure 15B shows cleavage at the N-terminal splice junction from MIP28 fusion. Purified protein samples were incubated at 4°C, 42°C, 50°C or 65°C for 1 hour. Products were analyzed by a 4/20% SDS-PAGE followed by Coomassie blue staining. Cleavage of the N-terminal splice junction of the MIP28 fusion protein (MIP) yielded MBP (M) and CIVPS-paramyosin ΔSal (IP). Size standards ( in kilodaltons) are shown on the left side.

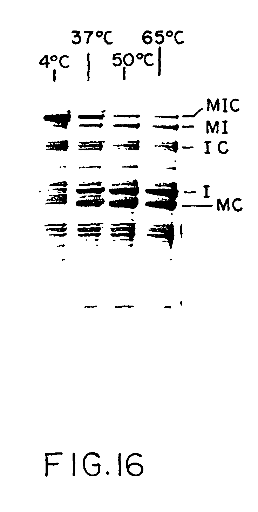

Figure 16 is a gel showing thermal inducible cleavage of MIC fusion. Purified fusion protein samples were incubated at 4°C, 37°C, 50°C or 650C for 1 hour. Products were analyzed by a 4/20% SDS-PAGE followed by Coomassie blue staining. Incubation of MIC fusion protein (MIC) yielded formation of ligated product, MBP-CBD(MC), and excised product, Deep-Vent IVPS1(I=I-Psp I). Also, cleavage products, MBP-Deep-Vent IVPS1(MI) and Deep-Vent IVPS1-CBD(IC), are present in all samples and do not change with this heat treatment.

### DETAILED DESCRIPTION OF THE INVENTION

The present invention is directed to modified proteins and methods of their production. The modified proteins comprise a controllable intervening protein sequence (CIVPS) and a target protein, the CIVPS being capable of excision by protein splicing, or cleavage in the absence of splicing, under predetermined conditions, e.g., increase in temperature, changes in pH conditions, unblocking of amino acid residues by photolysis, dephosphorylation, deglycosylation or other means. If desired, the modified protein can be subjected to these conditions. The CIVPS may also be inserted into a region that substantially inactivates target protein activity.

Intervening protein sequences (IVPS) are internal in-frame peptide segments found within a precursor protein which are removed or excised via protein splicing to form the native protein. IVPSs have been described in the *TFPI* allele from *Saccharomyces cerevisiae* [Hirata et al., *supra;* Kane et al., *supra*] and *rec A* gene from *Mycobacterium tuberculosis* [Davis et al., *supra (1991);* Davis et al., *supra* (1992)].

CIVPSs of the present invention include any intervening protein sequence in which excision or cleavage can be controlled, either by inherent properties of the native IVPS, such as an increase in temperature, or by modifications made to an IVPS that allow the reaction to be controlled.

The Vent DNA polymerase gene from the hyperthermophilic archaea *Thermococcus litoralis* contains two in-frame IVPSs, IVPS1 (CIVPS1) and IVPS2 (CIVPS2), [Perler, et al. *supra*] that can be deleted at the DNA level without affecting the kinetic and biochemical properties of the expressed polymerase. Correct processing of the Vent DNA polymerase gene containing both IVPSs occurs in the native archaea, *T. litoralis*. In addition, correct processing of expression constructs lacking IVPS1 has been observed in eubacterial *E. coli* [Perler, et al., *supra*], in eukaryotic baculovirus-infected insect cell and *in vitro* transcription/translation systems [Hodges, et al., *Nucleic Acids* *Research,* 20:6153 (1992)]. Furthermore, rabbit reticulocyte and *E. coli* in vitro transcription/translation systems correctly remove IVPS2 sequences to produce the mature polymerase. While not wishing to be bound by theory, it is believed that the Vent and Deep Vent IVPSs are self splicing.

The nucleotide sequence for the Vent DNA polymerase gene is set out in the Sequencing Listing as SEQ ID NO:1. The nucleotide sequence for CIVPS1 is from nucleotide 1773 to 3386. The nucleotide sequence for CIVPS2 is from nucleotide 3534 to 4703. CIVPS1 and CIVPS2 can be obtained from phage NEB 619, which was deposited with the American Type Culture Collection (ATCC) on April 24, 1990 and received ATCC accession number 40795.

A third IVPS (CIVPS3 or DV IVPS1), has been found by the present inventors in the DNA polymerase gene of the thermophilic archaebacteria, *Pyrococcus* species (isolate GB-D). The Pyrococcus DNA polymerase is sometimes referred to as Deep Vent DNA polymerase. The nucleotide sequence of the Deep Vent DNA polymerase is set out in the Sequence Listing as SEQ ID NO: 2. The nucleotide sequence for CIVPS3 is from 1839 to 3449. CIVPS3 can be obtained from plasmid pNEB #720 which was deposited with the ATCC on October 1, 1991 and received ATCC accession number 68723.

In accordance with the present invention, it has been found that the above CIVPS1, CIVPS2 and CIVPS3 are capable of excision from modified proteins upon an increase in temperature. For example, the CIVPSs are excised at reduced rates at temperatures from 37°C and below, but undergo excision more efficiently at temperatures from about 42°C to 80°C. Preferred excision temperatures are between about 42°C and 60°C. Most preferably, predetermined excision conditions are experimentally determined taking into consideration temperatures at which the target protein will not denature or undergo thermal inactivation. The modified proteins can be subjected to the predetermined temperatures for a period of time ranging from less than one minute to several hours. In certain situations, depending on the thermal sensitivity of the target protein, it may be desirable to increase the incubation time period while decreasing the temperature.

Additionally, different modified proteins may exhibit differences in splicing efficiency at various temperatures. If necessary, the optimum temperatures for isolation and splicing of each modified protein can be experimentally determined. If the CIVPS splices at too low a temperature for a proposed purpose, the CIVPS can be modified, or its position in the target protein changed such that the optimum splicing temperature is increased. If the optimum splicing temperature for a particular modified protein is about 37°C, in order to insure that the modified protein does not splice *in vivo*, and thus increase the yield of intact modified proteins, host cells can be grown and the modified protein purified at lower temperatures, e.g., 12°C-30°C. This can also be accomplished by mutating the splicing element to shift the splicing temperature optimum from, for example, 30-37°C to 42-50°C, and thus resulting in a reduced level of splicing at physiological temperature.

Other IVPSs can be isolated, for example, by identifying genes in which the coding capacity is significantly larger than the observed protein and that encodes a protein sequence not present in the mature protein. A protein containing an IVPS can be distinguished from a protein having a "pre-pro" precursor in that the mature protein will still have the N-terminal and C-terminal sequences of the IVPS containing precursor. Additionally, IVPSs can be detected by the absence of motifs that are conserved in certain protein families, e.g., DNA polymerases. The absence of such a motif may indicate that an IVPS is interrupting that motif [Perler et al., *supra*]. Suspected IVPSs can be screened by inserting the suspected protein sequence into a marker protein, e.g., β-galactosidase, such that the insertion decreases marker protein activity. The resulting modified protein can then be evaluated at periodic intervals for an increase in marker protein activity. See, Example 1-3. Once identified, the DNA encoding the IVPS can be isolated and manipulated using standard DNA manipulation techniques.

IVPSs may also be identified by a larger open reading frame than observed in the mature protein and the presence of a region which has some of the following properties: (1) similarity to HO endonuclease or other homing endonucleases, (2) the amino acid sequence (Ala/Val) His Asn (Ser/Cys/Thr) (SEQ ID NO:45).

CIVPSs of the present invention also include IVPSs which have been modified such that the splicing reaction can be controlled. As shown in Figure 1 (SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39), the aligned splice junctions of known protein splicing IVPSs reveal several similarities. In particular, -OH and -SH side chains are found on residues at the C-terminal side of both splice junctions, preceded by the dipeptide His-Asn at the downstream splice junction.

While not wishing to be bound by theory, it is believed that hydroxyl/sulfydryl groups participate in the splicing reaction and thus modification of these residues modulate the splicing reaction. Such modifications can be evaluated by inserting the modified CIVPS into a marker protein, e.g., β-galactosidase, such that the insertion decreases marker protein activity. The resulting modified protein can then be evaluated at periodic intervals and under controlled conditions for an increase in marker protein activity. See, Example 1-3. In addition, Western blot analysis can be used to evaluate splicing and cleavage products. See, Example 8. Once identified, the DNA encoding the CIVPS can be isolated and manipulated using standard DNA manipulation techniques.

In accordance with the present invention, it has been found that single amino acid changes at the serine 1082 of CIVPS2 slowed or blocked the protein splicing reaction. Specifically, the threonine substitution mutant displayed 10% of the polymerase activity of the wild-type enzyme, while the cysteine and alanine substitution mutants gave no detectable activity. However, a reaction product corresponding to cleavage at the altered splice junction was observed. This is species accumulated in a mutant which replaced the serine at the splice junction with cysteine, but was unaltered when serine was replaced with either threonine or alanine. Wild-type CIVPS2 showed accumulation of a species of the size expected for cleavage at the carboxy terminal splice junction during the splicing reaction, although accumulation of this product decreased, but was still observed, when serine 1082 was changed to threonine, cysteine, or alanine. The S1082A variant showed no evidence of protein splicing, but still produced this product.

Mutagenesis at the carboxy-terminal splice junction, namely amino acid substitutions for the threonine 1472 (T1472) residue with serine produced patterns of splicing identical to the wild-type. Replacement of T1472 with alanine, glycine, or isoleucine gave no detectable splicing. When asparagine 1471 was replaced with alanine, no splicing was observed, but evidence of cleavage at the amino splice junction was observed. Table 1, set forth below, summarizes the effects of amino acid substitutions on splicing and cleavage in CIVPS2.

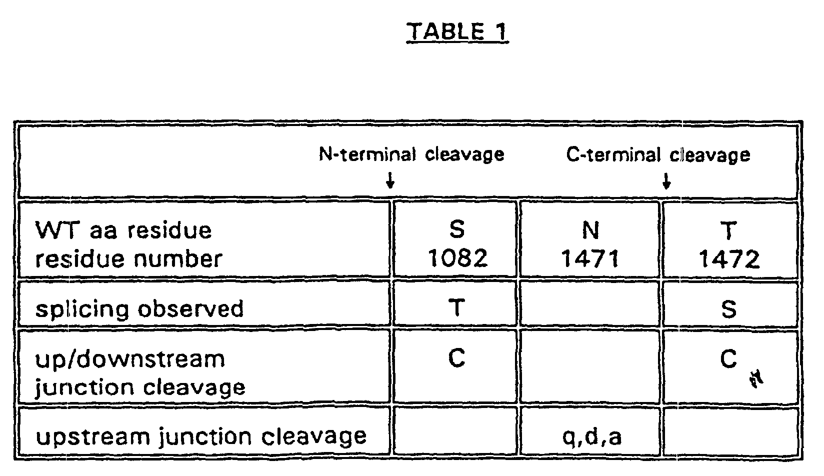

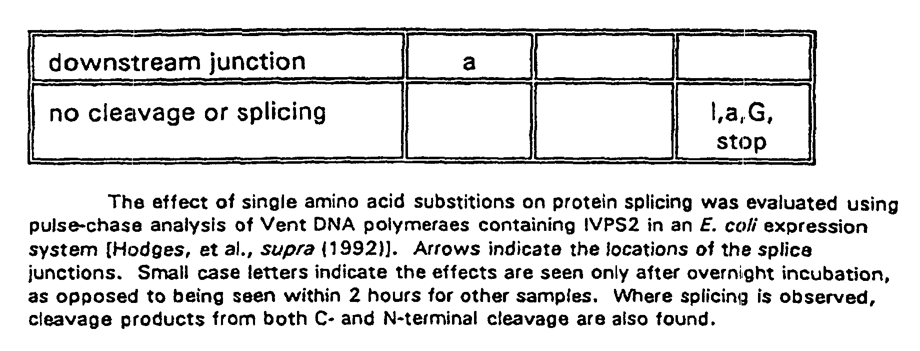

Accordingly, cleavage at the CIVPS splice junctions can be accomplished in the absence of protein splicing, thus allowing for controlled separation of the CIVPS from the target protein. In certain situations, such activity is desired. In these situations, the CIVPSs of the present invention may also encompass autoproteolytic proteins, such as autoproteolytic proteases, for example, retroviral proteases such as the HIV-1 protease [Louis, et al., *Eur. J. Biochem.,* 199:361 (1991)] and Debouck, et al., *Proc. Natl. Acad. Sci. USA,* 84:8903-8906 (1987)]. The skilled artisan is familiar with other such proteins. See, Kräusslich, et al., *Ann. Rev. Biochem.,* 701-754 (1988). Such proteins can be modified, in accordance with the disclosed methodology, such that the proteolytic activity is inducible under predetermined conditions.

Modification of the CIVPS amino acids, including splice junction amino acids, can be accomplished in a number of ways. For example, the sequence surrounding the amino acid residue to be modified may be altered to create a biological phosphorylation site allowing it to be a substrate for specific kinases and phosphatases. Examples of protein kinase include, for example, casein kinase II, cAMP-dependent protein kinase, cdc2, and pp60^{c-arc} [Pearson and Kemp, *Methods in Enzymology* 200:62 (1991)]. Examples of phosphatases include, for example, protein phosphatase 2A, lambda phosphatase, and the yop phosphatase from Yersinia [Tonks, *Current Opinion in Cell Biology*, 2:1114 (1990)].

Using CIVPS2 as an example, as set forth in Example 6C, an arginine residue was placed at position 1079 to create a consensus Calmodulin-dependent protein kinase II site [XRXXS*; Pearson et al., *supra*] The protein splicing reaction may then be regulated by the degree of phosphorylation, using a kinase to create phosphoserine and block the splicing, and phosphatases to remove the phosphate, restoring the wild type serine and, consequently, protein splicing.

Additionally, critical splice junction residues can be modified chemically such that the splicing reaction is blocked until the modification is reversed. This can be accomplished by using, for example, unnatural amino acid mutagenesis [Noren, et al., *Science* 244:182 (1989); Ellman, et al., *Methods in Enzymology* 202:301 (1991)]. Using this method, one of the amino acids involved in the splicing reaction can be replaced, during translation, by a synthetic derivative in which the side chain functionality of the side chain is "masked" by a chemically or photolytically removable group. For example, as set forth in Example 7, serine 1082 of CIVPS2 was modified by this method as follows: An amber stop codon was introduced into the Vent polymerase gene at the position corresponding to serine 1082 (see Example 6D). This gene was then added to an *in vitro* transcription/translation system [Ellman, et al., *supra*] that had previously been demonstrated to support protein splicing of the wild-type gene. In the absence of a tRNA to read through this codon, only truncated product was expected. When an amber suppressor tRNA that had been chemically aminoacylated with O-(o-nitrobenzyl) serine was added to the system, translation was able to continue past this codon, resulting in site-specific incorporation of the modified serine. As expected, only full-length precursor was observed, indicating that the splicing reaction was blocked (Figure 9). The o-nitrobenzyl group is removable by brief irradiation at 350 nm [Pillai, *Synthesis* 1 (1990)], so the blocked precursor would be expected to splice normally following irradiation. When the blocked precursor was exposed to visible light to free the serine and then incubated to allow the splicing reaction to occur, spliced product was clearly seen (Figure 10).

This strategy could also be applied to threonine 1472, which is found at the downstream splice junction of CIVPS2, as well as any other residue in which either the chemical functionality of the side chain is required for splicing, or introduction of a bulky group at that position would interfere with splicing sterically. Blocking groups can be chosen not only on the basis of the chemistry of the side chain to be protected, but also on the desired method of deblocking (chemically or photolytically). For example, the cysteine groups present in other examples of protein splicing (Figure 1) have thiol side chains that could be blocked using, for example, disulfide exchange (e.g., with dithiodipyridine) or complexation with transition metal ions (e.g., Hg²⁺). See, Corey and Schultz, *J. Biological Chemistry* 264:3666 (1989). The resulting blocked precursors could then be activated for splicing by mild reduction or addition of metal chelators, respectively.

It has been shown that IVPS1 and IVPS2 each encodes an endonuclease, I-Tli-II and I-Tli-I, respectively. In addition, DV IVPS1 also encodes an endonuclease, I-*Psp*I, which is inserted at the same position in the DV DNA polymerase gene as IVPS1 is in the Vent DNA polymerase gene and is 62% identical to the Vent IVPS1 gene. It has been found that the IVPS open reading frames in *Tfp 1*, *M*. *tuberculosis rec A*, Vent and Deep Vent DNA polymerase have protein sequence similarity to homing endonucleases, a class of intron-encoded proteins capable of cleaving alleles which lack the intron. [Hirata et al., *supra*, Kane et al., *supra*, Davis et al., *supra,* Perler et al., *supra*]

Certain host cells may not be able to tolerate the gene product of the CIVPS and thus, in some embodiments it may be preferable to inactivate the endonuclease function. In accordance with the present invention it has been shown that protein splicing can occur when the CIVPS endonuclease function has been inactivated. Such inactivation can be accomplished in a variety of ways, including for example, random mutagenesis, deletion or insertional inactivation, or site directed mutagenesis. Preferably, the endonuclease function is inactivated by site directed mutagenesis. I-Tli-I shares sequence similarity with other "homing endonucleases" in the pair of characteristic dodecapeptide motifs [Cummings et al., *Curr*. *Gent*. 16:381 (1989)]. As shown in Example 6B, endonuclease activity was inactivated by oligonucleotide-directed mutagenesis of a single residue (aspartate 1236 to alanine) within one of these motifs. Substitution of alternative residues could also reduce or abolish endonuclease activity without affecting protein splicing. Inactivation of endonuclease function has been shown to increase the stability of constructs carrying the modified proteins.

Target proteins which can be used in accordance with the present invention include, for example, enzymes, toxins, cytokines, glycoproteins and growth factors. Many such proteins are well known to the skilled artisan. The amino acid and nucleotide sequence of such proteins are easily available through many computer data bases, for example, GenBank, EMBL and Swiss-Prot. Alternatively, the nucleotide or amino acid sequence of a target protein can be determined using routine procedures in the art.

If it is desirable to substantially inactivate target protein activity, the CIVPS is inserted into a region(s) that will inactivate such activity. Such regions are well known to the skilled artisan and include, for example, binding sites, enzyme active sites, the conserved motifs of proteins, e.g., DNA polymerases, and dimerization or multimerization sites. Alternatively, the CIVPS may be inserted randomly and the activity of each modified protein measured until the desired level of activity is obtained. Preferably, such a modified protein has about a 50% reduced level of activity compared to the native protein. More preferably about 75%. Still more preferably greater than 99%.

The CIVPS may be inserted into the target gene by any number of means. Preferably, to assure proper protein splicing if the CIVPS is excised, it is important to insert the CIVPS immediately before a proper splice junction residue because excision of the CIVPS leaves that amino acid at the splice junction. This can be accomplished by either inserting the CIVPS immediately before the appropriate splice junction amino acid or by modifying the CIVPS such that it "brings" the appropriate amino acid with it.

For example, CIVPS1, 2 or 3 can be inserted immediately before the appropriate splice junction amino acids, for example, serine, threonine or cysteine residues, most preferably before serine or threonine. See, Figure 1. Such sites are readily available in most target proteins.

In certain situations, such as when the target protein is a toxin, it may be desirable to further control protein splicing by adding a secondary control. This may be accomplished by inserting the CIVPS before a less optimal amino acid, for example, one that the CIVPS does not normally precede and thus may slow down the splicing reaction.

As set forth above, insertion can be at any site within the target protein if the CIVPS "brings" the appropriate downstream amino acid with it. This can be accomplished by creation of CIVPS DNA having a codon for the desired downstream amino acid. Methods for producing such DNA are set out in detail below. This DNA can then be inserted at any site within the target DNA. Upon protein splicing of the resulting modified protein, the extra residue brought by the CIVPS will be left behind. Thus, if activity of the final product is important, the skilled artisan must takes steps to assure that the extra residue will not be left in an area of the target protein that will adversely affect activity.

The CIVPS may be inserted into the target protein, or fused to the target protein, by chemically synthesizing the primary amino acid sequence of the target protein, including the CIVPS, inserted at any desired site, using standard methods [e.g., see Hunkapiller, et al., *Nature* 310:105 (1984)] and a commercially available protein synthesizer.

Alternatively, a DNA sequence encoding a CIVPS is inserted in, or fused to, a DNA sequence encoding for a target protein such that both coding sequences form a continuous reading frame. This can be accomplished using a variety of methods known to the skilled artisan, several of which are set out below.

For example, the CIVPS DNA is inserted into any restriction enzyme site that makes a blunt cut in the target gene and which is in frame. This can be accompanied by first, synthesizing an CIVPS DNA fragment with a threonine codon (for Vent IVPS2) or a serine codon (for Deep Vent IVPS1 or Vent IVPS 1) at its 3' end. This fragment is then ligated in-frame to a linear plasmid cut to blunt ends by the restriction endonuclease. Using the *lac*Z DNA sequence, for example, an *Eco*RV site can be used to insert Vent IVPS2 or Deep Vent IVPS1 between residue 375 (aspartic acid) and 376 (isoleucine). See, Figure 2. However, as discussed above, using this method, if the CIVPS is excised an extra residue is expected to remain at the splice junction and therefore depending on where the CIVPS is inserted, the resulting protein may not have the same function or structure as the native protein.

The CIVPS DNA could also be inserted by making silent mutations (preserving the amino acid residue) near one end or both ends of the CIVPS to create restriction sites compatible with the target gene. Using CIVPS2 as an example, a *Bsp*EI restriction site can be made near the 5' end and a *Spe*I restriction site near its 3' end, by silent mutations. Using PCR primers overlapping the new restriction sites and continuing through the beginning of the *lac*Z target gene at either asp 594 or thr 595, one can generate a *lac*Z fragment with compatible *Bsp*E1 and *Spe*I restriction sites. Then, the CIVPS is inserted between an aspartic acid codon (residue 594) and a threonine codon (residue 595) within the *lac*Z coding region. DNA fragment(s) can be synthesized from both the CIVPS and the target gene by PCR with their ends at the insertion site overlapping with the termini of the CIVPS, therefore, including the same restriction sites. After appropriate restriction endonuclease treatment, DNA fragments with compatible ends can then be ligated to create a fusion gene. Since no extra residue would be left after excision of the CIVPS, native polypeptide will form when splicing occurs. Preferably, the restriction site being created is unique within the CIVPS and within the target gene to avoid ligation of multiple fragments and thus, complicated screening procedures.

If the plasmid vector carrying the target gene sequence is relatively small, for example, less than about 5Kb, a linear form of the plasmid can be generated using PCR, and then the linear plasmid can be ligated to the CIVPS gene. Using this method the CIVPS gene can be inserted at any location in the target gene as follows: First, plasmid DNA containing the target gene can be synthesized by PCR using a pair of primers starting at the insertion site, for example, serine or threonine codons for CIVPS1, 2 and 3, or any codon if the CIVPS also brings the appropriate downstream amino acid. Next, the CIVPS gene (with or without serine or threonine) can be ligated to the linear plasmid DNA (without the serine or threonine codon). The required splice junction amino acids (serine or threonine) can be positioned on either the CIVPS fragment or on the target gene. The advantage of having the required amino acid on the CIVPS fragment when placing upstream of an endogenous serine or threonine is that the self-ligated vector DNA (without the CIVPS insert) may only express a deficient product of the target gene because of the deletion of the serine or threonine in the coding region. This may aid in phenotype selection for the fusion construct if the fusion protein can splice to produce a functional product.

The fusion DNA encoding the modified protein can be inserted into an appropriate expression vector, i.e., a vector which contains the necessary elements for the transcription and translation of the inserted protein-coding sequence. A variety of host-vector systems may be utilized to express the protein-coding sequence. These include mammalian cell systems infected with virus (e.g., vaccinia virus, adenovirus, etc.); insect cell systems infected with virus (e.g., baculovirus); microorganisms such as yeast containing yeast vectors, or bacteria transformed with bacteriophage DNA, plasmid DNA or cosmid DNA. Depending on the host-vector system utilized, any one of a number of suitable transcription and translation elements may be used. For instance, when expressing a modified eukaryotic protein, it may be advantageous to use appropriate eukaryotic vectors and host cells. Expression of the fusion DNA results in the production of the modified proteins of the present invention.

Once obtained, the modified proteins can be separated and purified by appropriate combination of known techniques. These methods include, for example, methods utilizing solubility such as salt precipitation and solvent precipitation, methods utilizing the difference in molecular weight such as dialysis, ultra-filtration, gel-filtration, and SDS-polyacrylamide gel electrophoresis, methods utilizing a difference in electrical charge such as ion-exchange column chromatography, methods utilizing specific affinity such as affinity chromatography, methods utilizing a difference in hydrophobicity such as reverse-phase high performance liquid chromatography and methods utilizing a difference in isoelectric point, such as isoelectric focusing electrophoresis.

If desired, the modified proteins can be subjected to predetermined conditions under which the CIVPS is excised. Such conditions depend on the CIVPS used. For example, CIVPS 1, 2 and 3 are capable of excision by subjecting the modified protein to increased temperature, 42°C - 80°C, most preferably, 42°C - 60°C. This can be accomplished using any known means, for example a water bath or a heat generating laser. The time period for incubation can range from less than one minute to greater than several hours. As discussed above, in certain situations, depending on the thermal sensitivity of the target protein, it may be desirable to increase the incubation period while decreasing the temperature. In addition, if *in vivo* splicing is desired, temperatures compatible with the growth of the host organism are preferred.

The present invention may be used to produce proteins that are highly toxic to the host cells by using the CIVPS to modifying a toxic target protein such that the modified protein is non-toxic. This can be accomplished, for example, by inserting the CIVPS into a region(s) responsible for toxicity. After isolation, the non-toxic modified protein can then be subject to predetermined condition under which the CIVPS will excise and the resulting toxin can be isolated.

If a protein is extremely toxic to a host cell it may be desirable to produce that protein using a method referred to as "transplicing". Using this method the toxic protein is produced in two or more pieces in separate host cells, each piece being modified by insertion of a CIVPS. For example, a first modified protein can be produced comprising an amino portion of a target protein to which is inserted at its carboxy terminus an amino terminal fragment of a CIVPS, thereafter a second modified protein comprising the remaining portion of the target protein into which is inserted at its amino terminus the remaining fragment of CIVPSs. Alternatively, overlapping CIVPS fragments can be used. Each modified protein is then isolated from the host cells and incubated together under appropriate conditions for splicing of the CIVPS. This results in a ligated target protein. By dividing the target protein in two different hosts, there is no possibility that even a minute fraction will splice *in vivo,* adversely affecting the host. In addition, the entire CIVPS may be inserted on either side of the splice junction of the first modified protein and the remaining target protein fragment added to the splicing mixture.

The IVPSs of the present invention may be used in a "protein ligation" to add unnatural amino acid residues, structural probes, identifying epitopes or tags, or other determinants to a target protein. For example, the target protein can be fused to the amino terminus of the IVPS. A stop codon can be placed immediately following the carboxy terminus of the IVPS. The peptide to be fused can then be added to the mixture. If necessary, in order to more closely mimic the native splicing mechanism, the amino terminus of this peptide may be serine, threonine, or cysteine. The splicing reaction may then proceed, pushed by mass action towards splicing of the product.

The above reaction could also be adapted to occur with a starting material composed of the IVPS fused at the carboxy terminus to the amino terminus of the target protein. Initiation at a methionine engineered to precede the serine residue which begins in certain CIVPS would allow translation to occur which would likely be processed off in *E. coli* leaving an amino terminal serine residue. The peptide to be fused to the amino terminus of this target protein could then be added, and splicing allowed to proceed. Such an approach may be favored since there is no known requirement for the carboxy terminal residue on the peptide being added. Additionally, current experimental evidence suggests that cleavage of the upstream splice junction precedes the ligation reaction, indicating this approach more closely approximates the native reaction mechanism. Targeting peptides could also be added to the peptide to facilitate translocation of the fusion protein.

The present invention can also be used to study the effect of a target protein during a specific part of the cell cycle or under specific conditions such as induction of another protein or differentiation. For example, the chromosomal copy of a gene encoding a particular protein can be replaced with a version containing a CIVPS. At a specific point in the cell cycle, differentiation or other desired point, the cells are heated causing the precursor to splice, and thus the active target protein is present only at this point.

The CIVPs of the present invention can also be used to isolate modified proteins by use of affinity chromatography with antibodies specific to the CIVPS. For example, monoclonal or polyclonal antibodies can be generated having binding affinity to a CIVPS using standard techniques. These antibodies can then be utilized in affinity chromatography purification procedures to isolate a modified protein. After purification, if desired, the modified proteins can be subjected to predetermined conditions under which the CIVPS will undergo excision.

As discussed above, cleavage at the CIVPS splice junction can be accomplished in the absence of protein splicing, thus allowing for controlled separation of the CIVPS from the target protein. Such CIVPSs can therefore be used in a fusion protein purification system.

Fusion protein purification systems are well known to the skilled artisan. See, EPO 0 286 239 and N.M. Sassenfeld, *TIBTECH*, 8:88-93 (1990). Typically, in such systems, a binding protein and a target protein are joined by a linker having a protease recognition site. The fusion is then purified by affinity chromatography on a substrate having affinity for the binding protein. The binding protein and the target protein are then separated by contact with a protease, e.g., factor Xa. In these systems, in order to obtain a highly purified target protein, the protease must be separated from the target protein, thus adding an additional purification step, as well as the potential for contamination. The method of the present invention, by using a CIVPS, instead of a protease, avoids these and other problems encountered in currently used protein fusion purification systems.

In the method of the present invention, a modified protein comprising a fusion protein in which a CIVPS is between the target protein and a protein having affinity for a substrate (binding protein) is formed. Techniques for forming such fusion proteins are well known to the skilled artisan. See, EPO 0 286 239 and J. Sambrook, et al., Molecular Cloning: A Laboratory Manual (1989), Cold Spring Harbor Laboratory Press, Cold Spring Harbor, NY, p. 17.29-17.33.

Binding proteins which may be employed in the method of the present invention include, for example, sugar binding proteins, such as maltose or arabinose binding protein, receptor binding proteins, amino acids binding proteins and metal binding proteins. Other binding proteins are well known to the skilled artisan. See, EPO 0 286 239 and N.M. Sassenfeld, *TIBTECH*, *supra*.

The modified protein is then contacted with a substrate to which the binding protein has specific affinity, e.g., using affinity chromatography.

The highly purified target protein can be liberated from the column by subjecting the CIVPS to predetermined conditions under which cleavage is initiated, for example, between the CIVPS and the target protein. Alternatively, the purified fusion protein can be eluted from the column and liberated as above.

The present invention is further illustrated by the following examples. These examples are provided to aid in the understanding of the invention and are not construed as a limitation thereof.

### EXAMPLE 1

### SYNTHESIS OF IVPS CASSETTES FOR INSERTION INTO BLUNT SITES BETWEEN TARGET GENE CODONS

DNA fragments or cassettes for in-frame insertion of IVPSs into the *lac*Z coding region or any other target gene can be prepared by polymerase chain reaction (PCR) with or without the first downstream external protein sequence (EPS) codon. The native downstream residues are serine for Deep Vent IVPS1 and Vent IVPS1 or threonine for Vent IVPS2. It has been found that IVPS2 can splice if it precedes a threonine or cysteine, although at reduced levels. Although not wishing to be bound by theory, it is believed that all the IVPSs can splice to some extent when preceding either serine, threonine or cysteine. Cassettes including the downstream serine or threonine can be inserted at any desired location in the target gene including preceding a serine or threonine. In the latter constructions, one may delete the serine or threonine from the target gene and substitute it with the incoming residue on the cassette. Cassettes lacking downstream serines, threonines or cysteines may be inserted prior to a serine, threonine or cysteine in the target gene.

The following protocol describes the production of cassettes for Deep Vent IVPS1 (CIVP3) and Vent IVPS2 (CIVPS2) (endo⁺ and endo⁻ versions), including the first downstream EPS codon.

The PCR mixture contains Vent DNA polymerase buffer (NEB), supplemented with 2 mM Magnesium sulfate, 400 µM of each dNTP, 0.9 µM of each primer and 40 ng plasmid DNA and 2 units of Vent DNA polymerase in 100 µl. Amplification was carried out by using a Perkin-Elmer/Cetus thermal cycler at 94°C for 30 sec, 48°C for 30 sec and 72°C for 2 min for 30 cycles. Deep Vent IVPS1 was synthesized from pNEB #720 (ATCC No. 68723) which has a 4.8 Kb BamHI fragment containing the *Pyrococcus sp*. DNA polymerase gene inserted into the BamHI site of pUC19. Vent IVPS2 was synthesized from pV153-2 which has a 1.9 kb EcoR1 fragment (2851-4766) of the Vent DNA polymerase gene sequence in the vector Bluescribe SK- (Stratagene). Alternatively, pNEB671 (ATCC No. 68447) can also be used for IVPS2. pAMQ29 is an endonuclease-deficient derivative of pV153-2, carrying an amino acid substitution (aspartic acid 1236 to alanine) within the Vent IVPS2 coding region. Primers 5'-AGTGTCTCCGGAGAAAGTGAGAT-3' (SEQ ID NO:3) (Vent IVPS2 forward, 3534-3556, a substitution of A3542 to C) and 5'-AGTATT GTGTACCAGGATGTTG-3' (SEQ ID NO:4) (Vent IVPS2/Thr reverse, 4685-4706) were used to synthesize endo⁺ or endo⁻ Vent IVPS2 fragment (1173 bp) with a threonine codon at its 3' terminus. Primers 5'-AGCATTTTACCGGAAGAATGGGTT-3' (SEQ ID NO:5) (DV IVPS1 forward, 1839-1862) and 5'-GCTATTATGTGCATAGAGGAATCCA-3'(SEQ ID NO:6) (DV IVPS1/Ser reverse, 3428-3452) were used to synthesize the *Pyrococcus sp.* (or Deep Vent) IVPS1 fragment (1614 bp) with a serine codon at its 3' end. Reverse primers lacking the final three nucleotides could be used to generate IVPS fragments lacking the C-terminal serine or threonine.

The PCR samples were extracted with phenol and chloroform, precipitated in 0.3 M NaAc and 70% ethanol at -20°C for overnight, recovered by spinning at 10 K for 10 min in a microfuge, dried and each resuspended in 30 µl of distilled water, loaded on a 1 % agarose gel for electrophoresis at 60 volts for 15 hours. The gel slices that contain the PCR-amplified fragments were placed in a 1 % low melting agarose gel for electrophoresis at 80 volts for 2 hours. DNA fragments were recovered from the low melting agarose gel by incubation in 0.5 ml of TE buffer (10 mM Tris-HCI/0.1 mM EDTA, pH8.0) at 65°C for 30 min, extractions with phenol, phenol-chloroform (1:1 mixture) and chloroform, precipitation in 0.6 M NaAc (pH5.2) and 50% isopropanol at -20°C for overnight. DNA was spun down, washed with 70% ethanol, dried and resuspended in 15.5 µl distilled water.

Phosphorylation of the IVPS DNA fragments was performed at 37°C for 60 min with 2 µl of 10 x polynucleotide kinase buffer (NEB), 15.5 µl of purified DNA , 2 µl 10 mM ATP, and 5 units of T4 Polynucleotide kinase (NEB) in 20 µl. The samples were heated in a 65°C water bath for 10 min. After addition of 80 µl of TE buffer ( 10 mM Tris-HCl/0.1 mM EDTA, pH8.0), the samples were sequentially extracted with phenol, phenol-chloroform (1:1 mixture) and chloroform. DNA was precipitated in 2.5 M NH₄Ac and 70% ethanol at -70°C for 3.5 hours, pelleted by spinning at 10 K for 10 min in a microfuge, washed with cold 70% ethanol, dried and resuspended in distilled water (20 µl for Vent IVPS2 or Deep Vent IVPS1 DNA, 10 µl for Vent IVPS endo DNA).

### EXAMPLE 2

### IN-FRAME INSERTION OF IVPS IN A RESTRICTION ENZYME LINEARIZED PLASMID, SUCH AS ONE ENCODING β-GALACTOSIDASE

In this example, we describe how the IVPS cassettes can be cloned into a target gene by inserting the cassette at a restriction enzyme site which makes a blunt cut in the target gene between 2 codons. The cassette can carry a C-terminal serine, cysteine or threonine if necessary. This protocol works best if the restriction enzyme cuts the target gene vector once or twice. As an example, we describe insertion into the EcoRV site of the *lac*Z gene (Figure 2).

### PREPARATION OF ECORV-LINEARIZED PAHO5

pAHO5 carries the entire *lac*Z gene sequence on a 3.1 kb *Bam*HI-*Dra*I fragment from pRS415 (Simons, et al., *Gene* 53:85-96 (1987)) inserted between *Bam*HI and *Sma*I sites in the polylinker of pAGR3 downstream of a tac promoter. The tac promoter is a transcription control element which can be repressed by the product of the *lac*I^{q} gene and be induced by isopropyl β-D-thiogalactoside (IPTG). The 5.9 Kb vector pAHO5 (NEB) also has a transcription terminator sequence upstream of the tac promoter and the polylinker, and the *E*. *coli lac*I^{q} gene. pAHO5 contains two *Eco*RV recognition sequences. *Eco*RV leaves blunt ends at its cleavage site. One of the *Eco*RV cleavage sites cuts within the *lac*Z coding region between the 375th codon (aspartic acid) and the 376th codon (isoleucine) and is planned as the site for in-frame insertion of the IVPS fragments. The other site is located 3.2 Kb downstream within the *E*. *coli lac*I^{q} gene. The plasmid is cut partially to produce some molecules in which only one of the *Eco*RV sites has been cleaved. These linear plasmids are purified. The IVPS cassettes will be randomly cloned into either *Eco*RV site. Therefore, the resultant recombinants must be screened for orientation and insertion into the proper *Eco*RV site. DNA was partially digested by incubation of 15 µg of pAHO5 DNA with 40 units of *Eco*RV (NEB) in 100 µl of 1 x NEB buffer 2 at 37°C for 60 min. 20 µl agarose gel loading dye was added to the sample after the sample was heated to 65°C for 10 min to inactivate *Eco*RV. DNA fragments were separated by electrophoresis on a 1% low melting agarose gel. Linearized pAHO5 plasmid DNA was recovered from the low melting agarose gel as described in example 1 and resuspended in 44.6 µl of distilled water.

Dephosphorylation of *Eco*RV-linearized pAHO5 was carried out in 50 µl of 1 X NEB buffer 2 at 50°C for 60 min. in the presence of 2 µg DNA and 4 units of Calf Intestinal Alkaline Phosphotase (NEB). The sample was heated in a 65°C water bath for 30 min after addition of 0.5 µl of 0.5 M EDTA (pH8.0) and extracted with phenol, phenol-chloroform (1:1 mixture), and chloroform. DNA was precipitated in 0.75 M NH4Ac and 70% ethanol for 2 hours, recovered as described in Example 1, and resuspended in 20 µl of distilled water.

### CONSTRUCTION OF IVPS-lacZ FUSION GENES

Ligation of dephosphrylated pAHO5 DNA with phosphorylated IVPS fragments was carried out at 16°C for 15 hours in 20 µl volume with addition of 8.6 µl distilled water, 2µl of 10 X T4 DNA ligase buffer (NEB), 4 µl of 0.1 µg/µl dephosphorylated pAHO5 DNA, 5 µl IVPS DNA prepared as described above (0.25 µg of Vent IVPS2, 0.4 µg Deep Vent IVPS1 or 0.25 µg of Vent IVPS2 endo⁻) and 160 units of T4 DNA ligase (NEB).

*E*. *coli* strain RR1 was transformed by mixing 100 µl of competent RR1 cells with 10 µl of ligation sample on ice for 30 min., heating at 42°C for 2 min., chilling on ice for 5 min., adding 0.8 ml LB media (10 grams/liter tryptone, 5 grams/liter yeast extract, 10 grams/liter NaCl, 1 gram/liter Dextrose, 1 gram/liter MgCl₂·6H₂O, pH7.2 at 25°C) and incubating at 30°C for 45 min. The samples were plated onto LB plates, supplemented with 100 µg/ml ampicillin. After incubation overnight at 30°C, about 150-300 colonies per plate were observed.

Colony hybridization was utilized to screen for clones that carry recombinant plasmids. The Vent IVPS2 forward primer and the Deep vent IVPS1 forward primer, described in example 1, were radio-labeled with [Y-³²P] ATP using T4 polynucleotide kinase and used as hybridization probes. Colonies were lifted onto nitrocellulose and treated for 5 min. in each of the following solutions: 10% SDS, 0.5 M NaOH/1.5 M NaCl, 0.5 M Tris-HCI (pH7.5)/0.5 M NaCl (twice) and 2XSSC (twice). The nitrocellulose filters were dried at room temperature for 1 hour, baked in vacuum at 80°C for 2 hours, soaked in 6 x SSC for 5 min and washed in a solution of 50 mM Tris-CI (pH8.0), 1 M NaCI, 1 mM EDTA and 0.1% SDS at 42°C for 2 hours. After treatment at 42°C for 4 hours in 6 X NET, 5 X Denhardt's, 0.5% SDS and 25 µg/ml of denatured salmon sperm DNA, the filters were incubated with the radiolabeled oligomer probe under the same conditions for 16 hours and then washed in 6 x SSC at room temperature three times for 15 min, twice at 42°C for 2 min and twice at 50°C for two min, followed by autoradiogram. 36 clones were found to hybridize to the corresponding oligomer probes.

The positive clones were further analyzed to determine insert location by PCR amplification of plasmid DNA extracted from these clones, using the Vent IVPS2 forward primer (or the Deep Vent IVPS1 forward primer) described in Example 1, and a *lac*Z reverse primer (5'-AGGGTCGAC AGATTTGATCCAGCG-3' (SEQ ID NO:7)) complementary to the *lac*Z coding sequence (1417-1440, with a G:T mismatch at 1437) 392 nt downstream of the insertion site. PCR reactions from 14 clones produced the corresponding DNA fragments. Clones pVT133, 138, 139, 141, 142, and 144 contain the 1.4 Kb Vent IVPS2 insert, and pVTE 834, 836, 839 and 841 contain the Vent IVPS2 (endo⁻) insert, all yielding DNA fragments of approximately 1.1 kb. Clones pDVS 712, 742, 745 and 746 carry the 1.6 Kb Deep Vent IVPS1 insert, producing DNA fragments of about 2.0 Kb.

### EXPRESSION OF THE IVPS-lacZ FUSION GENES

The clones were further examined by their ability to express fusion (modified) proteins with inducer IPTG.

The clones were cultured in LB medium supplemented with 100 µg/ml ampicillin at 30°C until OD₆₀₀ₙₘ reached 0.5. To prepare lysate from uninduced cells, 1.5 ml of culture was pelleted and resuspended in 100 µl of urea lysis buffer, followed by boiling for 10 min. After addition of IPTG to a final concentration of 0.3 mM, the cultures were grown at 30°C for 4 additional hours. Cells from 1.5 ml culture were pelleted and then lysed with 250 µl of the urea lysis buffer after induction for 2 hours and 4 hours. Protein products were analyzed by Coomassie Blue stained gels. Three of the Vent IVPS2-*lac*Z fusion constructs (pVT139, 142 and 144) and all four Vent IVPS2 (endo⁻)-*lac*Z fusion constructs showed a major product of about 162-165 KDa, the expected size for a Vent IVPS2-β-galactosidase fusion protein. All four Deep Vent IVPS1-*lac*Z fusion clones expressed a larger product of 173-178 KDa, the expected size for the Deep Vent IVPS1-β-galactosidase fusion protein.

The identity of the Vent IVPS2 fusion proteins from pVT142 and 144, and pVTE836 and 839 was further analyzed by western blots using antibody raised against I-Tli-I (NEB) or β-galactosidase (Promega). Samples were electrophoresed on 4-20% SDS gels (ISS, Daichi, Tokyo, Japan) with prestained markers (BRL), transferred to nitrocellulose, probed with antisera (from mouse), and detected using alkaline phosphate-linked anti-mouse secondary antibody as described by the manufacturer (Promega). A band of approximately 160 KDa from all four clones being examined reacts with both sera and migrates at the same location as the Coomassie Blue stained band. Deep Vent IVPS1 fusions were also examined. Western blot analysis of pDVS712 and 742 using sera against β-galactosidase and I-*Psp*l (the protein product of Deep Vent IVPS1) yielded the predicted major band at about 168-175 KDa, identical to the Coomassie Blue stained band.

### EXAMPLE 3

### THERMAL CONTROL OF PROTEIN SPLICING IN β-GALACTOSIDASE-IVPS FUSIONS

The constructs described above (IVPSs inserted into the *lac*Z *Eco*RV site) yield fusion (modified) proteins after induction. The IVPS protein can be excised from the fusion protein to generate a ligated target protein (active β-galactosidase) and free IVPS endonuclease by incubation at elevated temperatures.

### SPLICING IS CONTROLLABLE BY TEMPERATURE INDUCTION: β-GALACTOSIDASE ACTIVITY IN CRUDE EXTRACTS INCREASES IN RESPONSE TO TEMPERATURE SHIFT

Crude extracts were prepared from cultures of RR1 (the *E. coli* host) and RR1 containing pAHO5 (the non-fusion β-galactosidase parent plasmid described in Example 2) or the fusion constructs, pVT142 (Vent IVPS2 or CIVPS2), pVTE836 (Vent IVPS1 endo⁻) or pDVS712 (Deep Vent IVPS1 or CIVPS 3) by the following steps. A single colony was inoculated in 10 ml LB medium supplemented with 100 µg/ml ampicillin, incubated at 30°C overnight, subcultured in 1 liter LB medium (100 µg/ml ampicillin) at 30°C to OD₆₀₀ₙₘ about 0.5 and induced with IPTG at 0.3 mM at 30°C for 2 hours. Cells were spun down and resuspended in 100 ml of LB, sonicated for 3 min at 4°C and spun at 7000 rpm for 15 min. The supernatants were recovered and stored at - 20°C.

7.5 ml aliquots of crude extracts were incubated in 42°C or 50°C water baths; 1 ml aliquots were taken at 1, 2 and 12 hours for pVT142 and pVTE836 extracts or 0.5, 1, 2, 4 and 16 hours for pDVS712, pAHO5 or RR1 extract.

β-galactosidase activity was measured according to Miller et al. [Experiments in Molecular Genetics (1972), Cold Spring Harbor, New York, Cold Spring Harbor Laboratory]. Assay buffer was prepared by mixing Z buffer with 2.7 µl/ml of 2-mercaptoethanol. Substrate o-nitrophenyl-β-D-galactopyranoside (ONPG) was dissolved in the assay buffer at 4 mg/ml. 0.1 ml of treated or untreated extract was transferred into a test tube containing 0.9 ml of assay buffer and 1 drop of 0.1%SDS and incubated for 5 min at 28°C. 0.1 ml LB medium was used for blank. 0.2 ml of 4 mg/ml ONPG was added to start an assay reaction. When adequate yellow color developed, the reaction was stopped by addition of 0.5 ml of 1 M Na₂CO_{3.} The incubation time was recorded and activity was measured on a spectrophotometer at OD₄₂₀ₙₘ and OD₅₅₀ₙₘ. The enzymatic activity from the heat-treated extract was calculated as follows. The activity after incubation was divided by the activity of the zero time point; the ratio was then multiplied by 100 to yield a percentage. Comparison of enzymatic activity indicated that while heat treatment had no effect on activity from RR1 or RR1/pAHO5 extract in the first two hours of incubation, all three IVPS-LacZ fusion constructs, pVT142, pVTE836 and pDVS712, exhibited an increase in enzymatic activity in response to the temperature shift to 42°C from 143% to 221% of untreated samples (Figure 3). This increase in β-galactosidase activity was due to excision of the IVPS and ligation of the two halves of β-galactosidase, forming more enzyme which was active. The splicing was confirmed by Western blot analysis. β-galactosidase activity in RR1 cells comes from expression of the chromosomal gene. The overnight incubation resulted in lower enzymatic activity from all samples, probably due to thermal inactivation of β-galactosidase (Figure 3).

### SPLICING IS CONTROLLABLE BY TEMPERATURE INDUCTION: ANALYSIS OF PROTEINS BY COOMASSIE BLUE STAINING AND WESTERN BLOTS

Analysis of IVPS-*lac*Z fusion protein synthesis in RR1 cells is complicated by chromosomal expression of β-galactosidase. Therefore, for ease of analysis, all the constructs were transferred to an *E. coli* host which did not synthesize β-galactosidase.

Preparation of crude cell extracts from the IVPS-*lac*Z fusion clones and western blot analysis of heat-treated samples were performed as followings.

The fusion constructs and the *lac*Z expression vector pAOH5 were introduced into a *lac*Z-deletion *E*. *coli* strain ER2267 (NEB) by the standard transformation procedure as previously described.

The cultures of ER2267 (50 ml), ER2267/pAHO5 (50 ml), pVT142 or pDVS712 plasmid (each in 1 liter) were grown at 30°C in LB media, supplemented with ampicillin at 100 µg/ml for plasmid-containing cells. When OD₈₀₀ₙₘ reached between 0.48 and 0.55, inducer IPTG was added into the cultures to 0.3 mM final concentration and the cultures were incubated at 23°C for 3 additional hours. Cells were spun down, resuspended in 50 ml (for ER2267 or pAH05-bearing ER2267) or 100 ml (for pVT142- or pDVS712-bearing ER2267) LB media, sonicated for 3 min at 4°C and spun at 7000 rpm for 10 min. The supernatants were stored at -20°C. Three 5 ml aliquots of each extract were incubated and sampled at 23°C, 42°C or 50°C for 16 hours. Aliquots of 0.9 ml were transferred into 1.5 ml microfuge tubes after incubation for 1, 2, 3, 4, 6 hours. 5 µl of untreated or treated extract was mixed with 10 µl of water and 5 µl of 5 x sample buffer (0.31 M Tris-Cl, pH6.8/10%SDS /25% 2-mercaptoethanol /50% glycerol/0.005% Bromophenol blue) and boiled for 10 min.

5 µl of each sample was loaded on a 4/20% SDS polyacrylamide and electrophoresed at 100 volts for 3-4 hours. Western blots, using antibody raised against β-galactosidase (Promega) and antibody raised against endonuclease I-Tli-I or I-*Psp*I (NEB), were carried out according to the procedure of Promega. The results showed barely trace amounts of endonuclease present in cells after IPTG induction at 23°C from both pVT142 and pDVS712 constructs, indicating inefficient excision activity, if any. However, after shifting the ER2267/pVT142 extract to higher temperatures, 42°C or 50°C, abundant IVPS2 product (I-Tli-I about 42 KDa), identical to the excised endonuclease from the Vent DNA polymerase precursor, was accumulated (Figure 4). A similar pattern was observed for pDVS712/ER2267 extract treated at 42°C or 50°C (Figure 4), resulting in accumulation of a product of about 60 KDa, expected for the Deep Vent IVPS1 product, I-*Psp*l.

Western blot analysis using antibody against β-galactosidase. indicated that excision of the IVPS domains was coupled with ligation or rejoining of the N-domain and the C-domain of the interrupted β-galactosidase. The heat-treated samples of both fusion constructs contained a product of 114 KDa, identical in size to full-length β-galactosidase (Figure 4).However, this product was only accumulated in small amount in the samples of pVT142, indicating that splicing from this fusion protein is inefficient under these conditions.

The fusion proteins were further tested for their ability to splice at higher temperatures, up to 80°C. The initial reaction rates at different temperatures were compared. The extracts were incubated in 300 µl aliquots in 1.5 ml-microfuge tubes at 42°C, 50°C, 65°C or 80°C. 20 µl were taken from each heated extract sample at 15 and 30 min and 1, 2, and 4 hours, and mixed with 40 µl of water and 20 µl of 5 x sample buffer and boiled for 10 min. Western blot analysis showed that Deep Vent IVPS-β-galactosidase fusion protein was able to splice at 65°C and at 80°C, although splicing seems more efficient at 65°C as measured by the accumulation of the 114 KD product. Excision of the Vent IVPS2 was efficient at 65°C but seems blocked at 80°C. Lack of accumulation may be due to thermal denaturation and precipitation of β-galactosidase at 80°C with time.

### EXAMPLE 4

### IN-FRAME INSERTION OF IVPS IN A PCR GENERATED LINEAR PLASMID, SUCH AS ONE ENCODING β-AGARASE I

In Example 2, we described inserting the IVPS cassettes from Example 1 into a restriction enzyme linearized plasmid. This method is limited by the availability of appropriate restriction enzyme sites in a target gene. PCR amplification using opposing primers on a circular plasmid allows linearization of any plasmid at any position, limited only by the capacity of the PCR reaction. Once the target plasmid is linear, the process is essentially the same as described in Example 2 for restriction enzyme generated linear plasmids.

As described in Example 2, insertion of an IVPS cassette into a target gene can be accomplished by ligation of an IVPS fragment with linear plasmid. In this example, PCR primers are used to generate plasmids linearized just prior to a serine or threonine codon. Thus, when the IVPS is excised and the two halves of the target protein are ligated, no extra amino acid is left behind in the target protein. The serine or threonine at the insertion site can be positioned on either the IVPS fragment or on the target gene fragment. If the serine or threonine is present on the IVPS cassette, then the target gene PCR primer can be constructed with a deletion of the 3 nucleotides encoding the first residue of the downstream EPS. If the IVPS cassette lacks the serine or threonine codon, then PCR with opposing, abutting PCR primers is used to synthesize target plasmid linearized at the serine or threonine codons.

This example describes cloning two IVPS elements, Vent IVPS2 and Deep Vent IVPS1, into a gene encoding β-agarase I [Yaphe, W., *Can. J*. *Microbiol*. 3:987-993 (1957)] by the procedure described in Example 2. The Deep Vent IVPS1 is inserted in front of a serine, the 108th codon, of the 290 amino acid β-agarase I gene, while the Vent IVPS2 is inserted in front of a threonine, the 133th codon of the β-agarase I gene.

The IVPS DNA fragments, including the serine codon (for Deep Vent IVPS1) or the threonine codon (for Vent IVPS2) at the 3' end, were prepared as described in Example 1. pAG6a1 (NEB), a 3.8 Kb recombinant plasmid containing the β-agarase I gene sequence in vector pUC18 in the orientation of lac promoter, was used as a PCR template to synthesize linear plasmid DNA fragments. Primers agaS108.rv (5'-GAGAACTTTGTTCGTACCTG-3' (SEQ ID NO:8)) and agaS108.fw (5'-GGTATTATTTCTTCTAAAGCA-3' (SEQ ID NO:9)) are compementary to DNA sequence 5' and 3' of the 108th codon, respectively. Primers agaT133.rv (5'-GTTGTTTGTTGGTTTTACCA-3' (SEQ ID NO:10)) and agaT133.fw (5'-ATGGCAAATGCTGTATGGAT-3' (SEQ ID NO:11)) are complementary to sequence 5' and 3' of the 133th codon, respectively. Each pair of the primers was used to synthesize linear plasmid DNA fragments, lacking the serine or threonine codon. The PCR mixture contained Vent DNA polymerase buffer (NEB), supplemented with 2 mM Magnesium sulfate, 400 µM of each dNTP, 0.5 µM of each primer, 20 ng plasmid DNA and 2 units of Vent DNA polymerase in 100 µl. Amplification was carried out using a Perkin-Elmer/Cetus thermal cycler at 94°C for 30 sec, 45°C for 30 sec and 72°C for 5 min for 30 cycles. The PCR samples were extracted with phenol and chloroform, precipitated in 0.3 M NaAcetate and 50% isopropanol, recovered by spinning at 10 Krpm for 10 min in a microfuge, dried and resuspended in 100 µl of distilled water. The DNA samples were then electrophoresed on a 1 % low melting agarose gel and PCR-synthesized fragments were recovered as described in Example 1.

Ligation of PCR-synthesized fragment with phosphorylated IVPS fragment (Example 1) was carried out at 16°C for 12 hours in 20 µl volume with addition of 9.5 µl distilled water, 2 µl of 10 X T4 DNA ligase buffer (NEB), 4 µl of 0.01 µg/µl PCR-synthesized plasmid DNA, 4 µl IVPS DNA (0.20 µg of Vent IVPS2 or 0.32 µg Deep Vent IVPS1) and 0.5 µl of 400,000 M/ml of T4 DNA ligase (NEB). Transformation of *E. coli* strain RR1 with the ligation samples was performed as described in Example 2. Transformants were cultured in LB medium, supplemented with 100 µg/ml ampicillin, for extraction of plasmid DNA using alkaline lysis method (Sambrook et al., Molecular Cloning: A Laboratory Manual (1989), Cold Spring Harbor Laboratory press, Cold Spring Harbor, New York). Plasmid DNAs were compared with pAG6a1 by electrophoresis on a 0.8% agarose gel followed by staining with ethidium bromide. Recombinant plasmid pAG108S18 contains the Deep Vent IVPS1 insert while pAG133T2β, 26, 31 and 35 all contain the Vent IVPS2 insert.

### EXPRESSION OF THE IVPS-β-AGARASE I FUSION GENES

The clones were further examined by their ability to express fusion proteins. RR1 cells carrying pAG108S18 or pAG133t35 were cultured in 1 liter of a modified LB medium, lacking dextrose, supplemented with 100 ug/ml ampicillin, at 30°C until OD₆₀₀ₙₘ reached about 0.5. After addition of inducer IPTG to a final concentration of 0.3 mM, the cultures were cooled down and grown at 25°C for 4 additional hours. Cells were spun down and resuspended in 50 ml LB medium. Crude extracts were prepared as described in Example 3. Western blots using antibodies raised against l-TIi-l (NEB), I-*Psp*l (NEB) and β-agarase I (NEB) were performed to detect fusion (modified) proteins expressed from these clones. Samples were electrophoresed on 4-20% SDS gels (ISS, Daichi, Tokyo, Japan) with prestained markers (BRL), transferred to nitrocellulose, probed with antisera (from mouse), and detected using alkaline phosphatase-linked anti-mouse secondary antibody as described by the manufacturer (Promega). Both anti-I-*Psp*I sera and anti-β-agarase I sera reacted with a 90-95 KDa product expressed from pAG108S18/RR1, of the expected size for a Deep Vent IVPS1 (approximately 60 KDa) - β-agarase I (approximately 30 KDa) fusion protein (Figure 5). Both anti-I-Tli-I sera and anti-β-agarase I sera reacted with a 70-75 KDa product,from pAG108S18/RR1, approximately the size expected for a Vent IVPS2 (42KDa)- β-agarase I fusion protein (Figure 5).

### EXAMPLE 5

### INSERTION OF IVPS INTO TARGET GENE BY CREATION OF NEW RESTRICTION ENZYMES SITES THROUGH SILENT SUBSTITUTIONS

In the previous examples, an IVPS cassette containing the entire IVPS sequence, with or without the first downstream EPS codon, was inserted into a blunt, linearized plasmid. It is also possible to create a restriction site by silent mutations (preserving the amino acid residue) near the ends of either the IVPS or the target gene.

### CREATION OF A RESTRICTION SITE NEAR THE END OF THE IVPS

It is possible to create a restriction site by silent mutations (preserving the amino acid residue) at both ends of an IVPS to facilitate insertion of the IVPS at any position within the target gene. After creation of the new restriction sites, the IVPS is cut with these enzymes. The target gene plasmid is generated by PCR. Since the restriction sites are within the IVPS, one must include the missing IVPS sequences on the 5' end of the respective target gene PCR primers to complete the IVPS and to generate compatible cloning sites in the target gene (Figure 6).

For example, silent mutations in Vent IVPS2 can create a *Bsp*EI site at the 5' end using primer Vent IVS2 Forward *Bsp*EI (5'-AGTGTCTCCGGAGAAAGTGAGAT-3' (SEQ ID NO:12)) and a *Spe*l at its 3' end, by using primer, Vent IVS2 Reverse *Spe*l (5'-ATTGTGTACTAGTATGTTGTTTGCAA-3' (SEQ ID NO:13)). It can then be inserted, for example, between an aspartic acid codon (residue 594) and a threonine codon (residue 595) within the *lac*Z coding region. A linear target gene plasmid can be generated by PCR as described in Example 4 with primers which include the *Bsp*EI and *Spe*l sites, the remaining portion of the IVPS and a region with identity to *lac*Z using primer, *lac*Z1/*Bsp*E1 reverse (5'-GCCTCCGGAGACACTATCGCCAAAATCACCGCCGTAA-3'(SEQ ID NO:14)) and primer, *lac*Z2/*Spe*I forward (5'-GCCACTAGTACACAATACGCCGAACGATCGCCAGTTCT-3'(SEQ ID NO:15)). DNA fragments are synthesized from both the IVPS and the target gene by PCR. Both IVPS and target gene primers contain the new restriction sites. After cutting with the appropriate restriction endonucleases, DNA fragments with compatible ends can then be ligated to create a fusion gene. Since no extra residue would be left after excision of the IVPS, native β-galactosidase polypeptide would be expected to form if splicing occurs.

Insertion of IVPS at restriction sites near the insertion site.

In another general approach (Figure 7), a restriction site near the insertion site in the target gene (for example, a threonine or a serine codon), can be used to insert an IVPS with ends compatible to the target gene. Restriction site(s) can be created by silent nucleotide substitution at or near the insertion site or native restriction sites can be used. A linear target gene plasmid is made by PCR as described in Example 4, beginning at the restriction sites near the insertion site. The IVPS is synthesized with primers containing the compatible restriction sites and the remainder of the target gene sequence (the sequence between the restriction site and the insertion site). The IVPS DNA fragment, with the ends overlapping the sequence at the insertion site, can be synthesized, cut with the appropriate enzyme(s), and then ligated to the vector that is cut by the same enzyme(s).

For example, IVPS elements can be inserted between residue 479 (aspartic acid) and 481 (serine) within the *lac*Z gene by creating a *Sal*I site (residues 478-479) and a *Xba*l site (residues 481-482 serine-arginine) by silent mutations. This can be achieved by PCR of the target plasmid, pAHO5, described in example 2, using primers, *lac*Z3 Sal reverse (5'-AGGGTCGACAGATTTGATCCAGCG-3' (SEQ ID NO:7)) and *lac*Z4 Xba forward (5'-CCTTCTAGACCGGTGCAGTATGAAGG-3' (SEQ ID NO:16)). Next the IVPS2 fragment is generated by PCR using primers, Vent IVS2 Forward *Sal*I (5'-GCCGTCGACCCTAGTGTCTCAGGAGAAAGTGAGATC-3' (SEQ ID NO:17)) and Vent IVS2 reverse *Xbal* (5'-GCCTCTAGAATTGTGTACCAGGATGTTGTTTGC-3' (SEQ ID NO:18)). DNA fragments are synthesized from both the IVPS and the target gene by PCR. Both IVPS and target gene primers contain the new restriction sites. Unfortunately, this vector also contains single *Xba*l and *Sal*I sites (Figure 7). Therefore, the target gene vector PCR product must be cut under conditions which produce partial digestion. The required linear plasmid is then isolated from agarose gels. After cutting with the appropriate restriction endonucleases, DNA fragments with compatible ends can then be ligated to create a fusion gene. Since no extra residue would be left after excision of the IVPS, native β-galactosidase polypeptide would be expected to form if splicing occurs. Generally, it is important to select or create an unique site within the target gene and vector to facilitate the cloning process as described above.

### EXAMPLE 6

A. To facilitate experimentation on the splicing of IVPS2 in Vent DNA polymerase, a modified version of the T7 promoter construct pV174-1B1 was created. This modified version, pANG5 (Figure 8), encodes a Vent DNA polymerase precursor identical to that of pV174-1B1. Numerous silent mutations were introduced to simplify the generation of mutants as discussed in thiis application, particularly at the upstream and downstream splice junctions. Changes included:
   1. Destroying *Xma*l and *Ppu*MI sites in the vector backbone. The *Xma*l site was removed first by cutting the T7 expression vector pAII17 with *Xma*I, repairing the cohesive ends with the Klenow fragment of DNA polymerase 1, and then religating the blunt termini. Plasmids were screened for resistance to cleavage by *Xma*l. The *Ppu*MI site was similarly removed from the resulting vector, screening this time for resistance to *Ppu*MI cleavage. The final vector was named pAML1. This vector allowed the use of unique *Xma*l and *Ppu*MI sites within the polymerase gene.
   2. Introduction of silent base changes to create restriction sites. Changes were introduced using oligonucleotide-directed mutagenesis as described by Kunkel [T.A. Kunkel, J.D. Roberts and R.A. Zakour, *Methods in Enzymology* 154:367-382 (1987)]. Single-strand templates were created in two Bluescript SK-phagemid derivatives by superinfection with the f1 helper phage IR1 [Enea, et al., *Virology* 122:22-226 (1982)]. The first contained a *Bsa*AI to *Bam*HI fragment (representing nucleotides 3714-5837 of the Vent DNA polymerase sequence) from pV174-1B1 ligated into *Bam*HI/*Eco*RV cut Bluescript. The second fragment included a *Cla*I to *Ssp*l fragment (nucleotides 816-4408) ligated into *Cla*I/*Eco*RV cut Bluescript.

   The *Bsa*AI/*Bam*HI construct was mutagenized simultaneously with three oligonucleotides: 
5'-GCAAAGAACCGGTGCGTCTCTTC-3' (SEQ ID NO:19) (AgeI nt
4669-4674)
 
5′-AGCAACAGAGTTACCTCTTG-3′ (SEQ ID NO:20)
(amber1703ochre)
 
5'-CAGTTTCCAGCTCCTACAATGAGACCTACGAGC-3' (SEQ ID
NO:21) (D1236A)
 where modified bases are underlined, and changes are indicated in parenthesis. The oligonucleotide to create D1236A also included silent base changes to create a *Bsa*l site to assist in screening. The resulting isolate was named pAMN2.
   The *Cla*I/*Ssp*I construct was mutagenized simultaneously with four oligonucleotides: 
5'-GTAGTGTCGACCCCATGCGG-3' (SEQ ID NO:22) (SalI nt
3863-3468)
 
5'-CGTTTTGCCTGATTATTATCTCACTTTC-3' (SEQ ID NO:23)
(BsaBI nt 3554-3563))
 
5'-GTCCACCTTCGAAAAAAGATCC-3' (SEQ ID NO:24) (BstBI nt
3608-3613)
 
5'-CCGCATAAAGGACCTTAAAGC-3' (SEQ ID NO:25) (PpuMI nt
3517-3523)
 where markings are as above. Screening was also as above, with the resulting construct was named pAMO22.
   The *Bsa*AI/*Bam*HI construct was also mutagenized with the oligonucleotide: 
5'-GAGGAAGAGATCATCATCATAGC-3' (SEQ ID NO:26) (BsaBI
blocking nt 5641)
 and screened for resistance to *Bsa*BI cleavage due to the addition of a dam methylation site. The resulting construct was named pAMW3.
   Finally, the Ndel site at the initiation codon of pV174-1B1 was inactivated by partial *Nde*I cleavage, repairing the termini with Klenow, and recircularizing using T4 DNA ligase. Plasmids were screened for the loss of the appropriate *Nde*I site. One such construct was named pAKC4.
   The pANG5 construct was assembled from the above parts:
   1. *Xba*I/*Cla*I from pAKC4 (translation initiation and amino terminus of vent DNA polymerase)
   2. *Cla*I/*Nde*I from pAMO22 (more amino terminal polymerase plus the amino terminal region of IVPS2)
   3. *Nde*I/*Nsi*I from pAMN2 (carboxyl terminal region of IVPS2, carboxyl terminal region of vent DNA polymerase)
   *4. Nsi*I/*Bam*HI from pAMW3 (final 5 amino acids of the polymerase plus the downstream region)
   5. *Bam*HI/*Xba*I from pAML1 (T7 promoter, origin of replication, ampicillin resistance).

   Comparisons between pANG5 and the parent pV174-1B1 show identical patterns of Vent DNA polymerase and I-*Tli*I production, with the exception of the greater viability of the pANG5 containing strains, as discussed below. This is as expected if splicing occurs at the protein level, as opposed to at the RNA or DNA level.
B. During work on the expression of the Vent DNA polymerase gene in *E*. *coli* it was found that a large increase in expression and cell viability occurred after deletion of IVPS1 and IVPS2. This increase could either represent toxic effects of I-*Tli*II and I-*Tli*I, the gene products of IVPS1 and IVPS2, respectively, or toxic effects of the splicing reaction itself. It was reasoned that endonuclease and splicing activities could well be independent, allowing inactivation of the endonuclease without affecting splicing. A single amino acid substitution to A as described in the construction of pANG5 was made in a conserved residue within the amino-proximal dodecapeptide motif of I-*Tli*I (changed residue D1236). Although these constructs expressed Vent DNA polymerase, no I-*Tli*I activity was detected. Unlike pV174-1B1 T7 expression strains such as BL21 (DE3) tolerated pANG5 well, even at 37°C. Analysis of protein splicing by western blot and pulse-chase analysis showed no discernible differences in protein splicing between pANG5 and pV174-1B1, namely production of a full-length precursor and subsequent formation of the mature polymerase and a protein corresponding in size to I-*Tli*I.
C. A consensus calmodulin-dependent protein kinase II site (XRXXS*; Pearson et al., *supra)* was constructed, replacing tyrosine 1079 with arginine using cassette replacement mutagenesis. In short, pANG5 was cut at the unique sites *Bsa*BI and *Ppu*MI and the duplex (SEQ ID NO:27) listed below was inserted, introducing the desired change. 5'-GTCCTTCGTGCGGACAGTGTCTCAGGAGAAAGTGAGATAA-3' 3'- GAAGCACGCCTGTCACAGAGTCCTCTTTCACTCTATT-5' The correct construct was verified by DNA sequencing.
D. Introduction of an amber stop codon for adding a blocked amino acid was accomplished by cassette replacement mutagenesis in 
5′-GTCCTTTATGCGGACTAGGTCTCAGGAGAAAGTGAGATAA-3′
3GAAATACGCCTGATCCAGAGTCCTCTTTCACTCTATT-5′.
   Similarly, tyrosine 1472 was replaced with an amber termination codon by placing the following duplex (SEQ ID NO:29) into pANG5 cut with *Age*l and *Sma*l: 
5'-CCGGTTCTTTGCAAACAACATCCTGGTACACAATTAAGACGGC
3'-     AAGAAACGTTTGTTGTAGGACCATGTGTTAATTCTGCCG
 
TTTTATGCCACAATACCC-3′
AAAATACGGTGTTATGGG-5′
   Finally, since the Vent DNA polymerase gene ends in an amber codon (TAG), that termination codon will be changed to an ochre codon (TAA) by inserting an appropriate restriction fragment from pAMN2 (described above) into the corresponding site in pANG5.

### EXAMPLE 7

### CONTROL OF PROTEIN SPLICING BY INCORPORATION OF O-(O-NITROBENZYL) SERINE AT THE SPLICE JUNCTION OF CIVPS2

Two vectors were constructed using pV174.1B1 to demonstrate photoactivatable protein splicing. The first construct, pANY5 (also referred to as "wild-type"), can be described on the amino acid level as follows: pV174.1B1 Δ1-1063, Δ1544-1702, V1542M, V1541M, 1543opal(TGA). This construct is designed to give a 55.8 kDa precursor protein, which splices out the 45.3 kDa endonuclease (I-*Tli*I) and yields a 10.5 kDa ligation product, when translated in an *in vitro* transcription/translation system. The second construct, pAOD1 (also referred to as the "amber mutant"), can be described on the amino acid level as follows: pV174.1B1 Δ1-1063, Δ1544-1702, V1542M, V1541 M, 1543opal(TGA), S1082amber(TAG). This construct is designed to give a 2.2 kDa amber fragment under standard *in vitro* transcription/translation conditions, but will incorporate a photoactivatible serine when the *in vitro* reaction is supplemented with an amber suppressor tRNA that has been chemically aminocylated with o-nitrobenzylserine. With the serine at position 1082 "blocked", the precursor is unable to splice. When irradiated with intense 350 nm light, the o-nitrobenzyl group is released [Pillai, *supra*], the nuceophilic hydroxyl side chain of serine is freed, and the protein is able to splice.

The amber suppresssor tRNA (lacking the 3' terminal CA residues) was synthesized on milligram scale by *in vitro* runoff transcription of *Fok*I-linearized pYPhe2 plasmid template with T7 RNA polymerase as described [Ellman, et al., *supra;* Noren, et al., *Nucleic Acids Res*. 18:83 (1990)]. Serine derivatives protected at the a amine with functionalities like BPOC, CBZ, or BOC are available from commercial sources (Bachem, Sigma, Aldrich). N-blocked serine can be converted to N-blocked O-(o-nitrobenzyl) serine by a standard alkyl halide substitution reaction with a reagent such as o-nitrobenzylbromide. The fully blocked serine was then coupled to 5'-phosphodeoxyribocytidylyl-(3'-5')-riboadenosine (pdCpA) as described [Ellman, et al., *supra*]. The aminoacylated dimer was then ligated to the truncated suppressor tRNA with T4 RNA ligase (New England Biolabs, Inc.) to yield full-length aminoacylated suppressor tRNA.

*In vitro* transcription/translation of the "wild-type" construct was carried out by combining on ice: 3 µg cesium chloride-purified plasmid DNA, 3 µl 100 mM magnesium acetate, 1 µl 100 mM calcium acetate, 7.5 µl low molecular weight mix [Ellman, et al., *supra*] (no calcium or methionine), 1 µl [³⁵S]-methionine (10 µCi/µL, 1000 Ci/mmol), 1 µl 3 mg/ml rifampicin, and water to 30 µL. The reactions were incubated for 3 minutes at 37°C while an aliquot of S-30 extract prepared from *E. coli* D10 [Ellman, et al. *supra*] was thawed. 8.5 µl of S-30 extract was added, followed by 1.5 µl of T7 RNA polymerase (300 U/µL, New England Biolabs, Inc.), and the reactions were incubated 60 min. at 37°C. Samples were electrophoresed on a 10-20% tricine SDS-PAGE gel (NOVEX) and autoradiographed to visualize the proteins (Figure 9).

*In vitro* transcription/translation of the "amber mutant" was carried out as described for the "wild-type" except that the reactions werre supplemented with 3.5 µl of chemically aminoaceylated o-nitrobenzylsserine-tRNA_{amber} at a concentration of approx. 3 µg/µl. The suppressor tRNA was added to the reaction immediately before addition of the S-30 extract.

Figure 9 shows a 10-20% tricine SDS-PAGE gel of *in vitro* transcription/translation reactions primed with either the "wild-type (pANY5) or "amber mutant" (pAOD1) constructs. Lane 1 shows the 55.8 kDa precursor and excised 45.3 kDa I-*Tli*I endonuclease expressed *in vitro* from the "wild-type" construct. Lane 2 shows the "wild-type" reaction supplemented with 13.5 µg of full length uncharged amber suppressor tRNA to demonstrate there is no inhibition of translation due to added tRNA. Lanes 3 and 4 show the result of *in vitro* expression of the "amber mutant" without and with full length unacylated supressor tRNA (10.5 µg) added. Neither of these reactions produce the full length precursor molecule, nor any splice products, as expected. This indicates that the suppressor tRNA is not aminoacylated by any of the endogenous aminoacyl-tRNA synthetases in the cell extract. The band of approximate molecular weight 52 kDa is apparently caused by a secondary translational initiation site just downstream from the amber mutation. Lane 5 shows the result of supplementing the "amber mutant" with the chemically aminoacylated O-nitrobenzylserine-tRNA_{amber}. Precursor protein is produced *in vitro,* but no splice products (i.e., I-*Tli*I) are visible.

Controlled splicing was achieved by photochemically removing the o-nitrobenzyl group from the serine which had been incorporated site-specifically at position 1082 of the precursor protein. A 6 µL aliquot of an *in vitro* reaction was treated with 0.5 µl of RNase A (10 µg/µl) to arrest translation, irradiated with intense (275 W) visible light from a GE model #RSK6B tanning lamp at 10 cm for 10 minutes, diluted with 4 µl of water, and then incubated at 37°C for 60 minutes to allow splicing to occur. The resulting splice products were visualized by electrophoresis on a 10-20% tricine SDS-PAGE gel followed by autoradiography (Figure 10).

Figure 10 illustrates the results of exposing the chemically blocked precursor (Lane 5, Figure 9) to 350 nm light. Lanes 1 through 4 are controls in which the "wild-type" reaction (Lane 1, Figure 9) was treated as follows. Lane 1, incubated 60 min. at 37°C; Lane 2 added 0.5 µl RNase (10 µg/µl) and incubated 60 min. at 37°C; Lane 3, irradiated 10 minutes with 350 nm light and incubated 60 min. at 37°C; Lane 4, treated with RNase as above, irradiated 10 min. with 350 nm light and incubated 60 min. at 37°C. Lanes 5-8 show the result of treating the "blocked" precursor (Lane 5, Figure 9) in the same way as for Lanes 1-4, respectively. Irradiated of the "blocked" precursor results in the excision of the I-*Tli*I (45.3 kDa) endonuclease encoded by IVPS2 (cf. Lanes 7-8 with Lanes 5-6).

### EXAMPLE 8

### IN-FRAME INSERTION OF MODIFIED IVPS INTO A TARGET GENE AND THERMAL CONTROL OF PEPTIDE BOND CLEAVAGE

In this example, we describe how an IVPS (CIVPS) cassette can be modified and inserted into a target gene. As an example, we describe modification of *Pyrococcus sp*. (or Deep Vent) IVPS1 (CIVPS3) by substitution or deletion of the first native downstream residue (serine), and in-frame insertion of the modified cassettes into the *Eco*RV site of the *E*. *coli lac*Z gene.

### MODIFICATION OF IVPS CASSETTES

In general, an IVPS cassette can be modified by substitution and deletion of residue(s) or addition of residue(s) to one or both ends of IVPS. The modified or fusion proteins using such modified IVPS cassettes may exhibit different catalytic activities, such as splicing (peptide ligation) or cleavage at a specific peptide bond.

As previously discussed, the first downstream residues at the carboxyl splice junction are serine for Deep Vent IVPS1 (CIVPS3) and Vent IVPS1 or threonine for Vent IVPS2. The first IVPS residue at the amino splice junction of CIVPS1, CIVPS2 and CIVPS3 is serine. Cysteine residues have been found at the splice junctions of the yeast TFP1 and M. tuberculosis RecA (See, Hirata, et al., *supra*; Kane, et al., *supra*; Davis, et al., *supra*). It is believed that serine, threonine or cysteine residues at splice junctions are essential for protein splicing and cleavage. The previous examples have shown that an IVPS with the first downstream residue is sufficient to contain information for protein splicing. However, these residues may function differently in various IVPS contexts. Substitutions of the native residue, for example, a serine by threonine or cysteine in the Vent IVPS2 (CIVPS2) resulted in reduced splicing and altered cleavage activity (see, Hodges, et al., *supra*).

### SYNTHESIS OF MODIFIED IVPS CASSETTES FOR IN-FRAME INSERTION INTO BLUNT SITES BETWEEN TARGET GENE CODON

IVPS cassettes for in-frame insertion into the *lac*Z coding region or any other target gene can be prepared by polymerase chain reaction (PCR). The following protocol describes the production of four Deep Vent IVPS1 cassettes without or with an additional carboxyl terminal codon, serine, threonine or cysteine, referred as CIVPS3, CIVPS3/Ser, CIVPS3/Thr and CIVPS3/Cys, respectively.

Primer S'-AGCATTTTACCGGAAGAATGGGTT-3' (SEQ ID NO:5) (DV IVPS1 forward, 1839-1862) and one of the four reverse primers described below were used to synthesize the cassettes from pNEB#720 (ATCC No. 68723). pNEB#720 used as template has a 4.8 Kb *Bam*HI fragment containing Deep Vent DNA polymerase gene inserted into the *Bam*HI site of pUC19. Reverse primers 5'-GCAATTATGTGCATAGAGGAATCCA-3' (SEQ ID NO:40) and 5'-GGTATTATGTGCATAGAGGAATCCA-3' (SEQ ID NO:41) (3428-3452) were used to generate CIVPS3/Thr and and CIVPS3/Cys fragments (1614 bp), respectively. The PCR mixture contains Vent DNA polymerase buffer (NEB), supplemented with 2 mM magnesium sulfate, 400 µM of each dNTP, 100 µg/ml BSA, 0.9 µM of each primer and 40 ng plasmid DNA and 2 units of Vent DNA polymerase in 100 µl. Amplification was carried out by using a Perkin-Elmer/Cetus thermal cycler at 94°C for 30 sec., 48°C for 30 sec. and 72°C for 2 min for 20 cycles. Primer 5'-ATTATGTGCATAGAGGAATCCAAAG-3' (SEQ ID NO:42) (3425-3449) was used to synthesize CIVPS3 fragment (1611 bp) by PCR as described above except the amplification was carried out for 30 cycles. Primer 5'-GCTATTATGTGCATAGAGGAA TCCA-3' (SEQ ID NO:6) (3428-3452) were used to synthesize IVPS1/Ser fragment (1614 bp) as previously described in Example 1.

The PCR samples were extracted with phenol and chloroform, and precipitated in 0.3 µM NaAc and 50% isopropanol at -20°C for 6 hours, recovered by spinning at 10 Krpm for 10 min. in a microfuge, dried and each resuspended in 20 µl of distilled water, loaded on a 1 % low melting agarose gel for electrophoresis at 80 volts for 6 hours. DNA fragments were recovered from the low melting agarose gel by incubation in 0.4 ml of TE buffer (10 mM Tris-HCI/0.1 mM EDTA, pH 8.0) at 65°C for 30 min., extractions with phenol and chloroform, precipitation in 0.3 µM NaAc (pH5.2) and 50% isopropanol at -20°C for overnight. DNA was spun down, washed with 70% ethanol, dried and resspended in 10 µl distilled water.

Phosphorylation of the IVPS1 DNA fragments was performed at 37°C for 60 min. with 4 µl of 10 x polynucleotide kinase buffer (NEB), 31 µl of purified DNA, 4 µl 10 mM ATP, and 10 units of T4 polynucleotide kinase (NEB) in 40 µl. The samples were heated in a 65°C water bath for 10 min. After addition of 80 µl of TE bffer (10 mM Tris-HCl/0.1 mM EDTA, pH 8.0), the samples were sequentially extracted with phenol and chloroform. DNA was precipitated in 2.4 µM NH₄AC and 70% ethanol at -70°C overnight, pelleted by spinning at 10 Krpm for 10 min. in a microfge, washed with cold 70% ethanol, dried and resuspended in 20 µl distilled water. Phosphorylation of the CIVPS3/Ser fragment was as described above.

### IN-FRAME INSERTION OF CIVPS3 CASSETTES INTO THE ECORV SITED OF THE E. COLI lacZ GENE IN VECTOR pAH05

PCR-synthesized CIVPS cassettes can be inserted into a target coding region by ligation with linearized vector bearing the target gene. Linear plasmid vector can be prepared by restriction enzyme or PCR synthesis as previously described. pAH05 carries the entire *lac*Z gene sequence on a 3.1 kb *Bam*HI*-Dra*I fragment from pRS415 (Simons, et al., *Gene,* 53:85-96 (1987)) inserted between *Bam*HI and *Sma*l sites in the polylinker of pAGR3 (NEB) downstream of a tac promoter. The tac promoter is a transcription control element which can be repressed by the prodct of the *lac*I^{q} gene and be induced by isopropyl β-D-thiogalactoside (IPTG). pAH05 contains two *Eco*RV recognition seqences. *Eco*RV leaves blunt ends at its cleavage site. One of the *Eco*RV cleavage sites cuts within *lac*Z coding region between the 375th codon (aspartic acid) and the 376th codon (isoleucine).

DNA was partially digested by incubation of 15 µl of pAH05 DNA with 40 units of *Eco*RV (NEB) in 100 µl of 1 x NEB bffer 2 at 37°C for 60 min. 20 µl agarose gel loading dye was added to the sample after the sample was heated to 65°C for 10 min. to inactivate *Eco*RV. DNA fragments were separated by electrophoresis on a 1 % low melting agarose gel. Linearized pAH05 plasmid DNA was recovered from the low melting agarose gel as described in Example 8 and resuspended in distilled water.

### CONSTRUCTION OF CIVPS-lacZ FUSION GENES

Construction of CIVPS3/Ser-*lac*Z fsion was described in Example 2. CIVPS3-*lac*Z fusion was made by ligation of dephosphorylated pAHO5 DNA to the phosphorylated IVPS1 fragment. The reaction was carried at 16°C for 5 hours in 20 µl volume with 1X T4 DNA ligase bffer (NEB), 0.1 µg pAHO5 DNA, 0.5 µg IVPS1 DNA and 160 units of T4 DNA ligase (NEB). *E*. *coli* strain RR1 was transformed by mixing 100 µl of competent RR1 cells with 10 µl of ligation sample on ice for 30 min., heating at 42°C for 2 min., chilling on ice for 5 min., adding 0.8 ml LB media (10 grams/liter tryptone, 5 grams/liter yeast extract, 10 grams/liter NaCI, 1 gram/liter dextrose, 1 gram/liter MgCl₂6H₂O, pH 7.2 at 25°C) and incubating at 30°C for 45 min. The samples were plated onto LB plates, supplemented with 100 µg/ml ampicillin. After incubation overnight at 30°C, abot 150-300 colonies per plate were observed.

CIVPS3/Thr-*lac*Z and CIVPS3/Cys-*lac*Z fusions were made by ligation of 0.1 µg *Eco*RV-linearized pAH05 DNA with 0.7 g of CIVPS3/Thr or CIVPS3/Cys fragment. Transformation of *E*. *coli* strain ER2252 (NEB) was carried out by the same protocol as described above.

Colony hybridization was utilized to screen for clones that carry recombinant plasmids. The Deep Vent CIVPS3 forward primer, described above, was radio-labeled with T4 polynucleotide kinase and used as a hybridization probe. Colonies were lifted onto nitrocellulose and treated for 5 min. in each of the following soltions: 10% SDS, 0.5 M NaOH/1.5 M NaCl, 0.5 M Tris-HCI (pH 7.4)/0.5 M NaCl (twice) and 2XSSC (twice). The nitrocellulose filters were dried at room temperature for 1 hour, baked in vacuum at 80°C for 2 hours, soaked in 6 x SSC for 5 min. and washed in a solution of 50 mM Tris-HCI (pH 8.0), 1 M NaCI, 1 mM EDTA and 0.1% SDS at 42°C for 2 hours. After treatment at 42°C for 4 hors in 6 X NET, 5 X Denhardt's, and 0.5% SDS, the filters were incubated with the radiolabeled oligomer probe under the same conditions for overnight and then washed in 2 x SSC for times at 42°C for 15 min. and twice at 50°C for two min., followed by autoradiography.

The positive clones that hybridized to the oligomer probe were further examined by their ability to express fusion proteins with inducer IPTG. The clones were cultured in LB medium supplemented with 100 µg/ml ampicillin at 30°C until OD₆₀₀ₙₘ reached 0.5. After addition of IPTG to a final concentration of 0.3 mM, the cultures were grown at 30°C for 4 additional hours. Crude lysates were prepared by boiling 0.1 ml of cells with 0.1 ml of the urea lysis buffer for 10 min. The identity of the fusion proteins from the positive clones described above was analyzed by Western blots using antibody raised against β-galactosidase (Promega) or I*-Psp*l (the protein product of Deep Vent CIVPS3, NEB). Samples were electrophoresed on 4-20% SDS gels (ISS, Daichi, Tokyo, Japan) with prestained markers (BRL), transferred to nitrocellulose, probed with antisera (from mouse), and detected using alkaline phosphate-linked anti-mouse secondary antibody as described by the manufacturer (Promega). Deep Vent CIVPS3-*lac*Z fusion clones expressed a product, reacting with both antibodies, of 173-178 KDa, the expected size for the CIVPS3-β-galactosidase fusion proteins (Figre 11). Clones pDV7 and pDV15 contain CIVPS3 insert. pDVC302, 306 and 307 carry the CIVPS3/Cys cassette while pDVT319, 321, 322 and 323 contain the CIVPS3/Thr cassette. pDVS712 and 742 containing the CIVPS3/Ser insert were previously described in Example 2.

### THERMAL CONTROL OF SPECIFIC PEPTIDE BOND CLEAVAGE IN CIVPS3-β-GALACTOSIDASE FUSIONS USING MODIFIED CIVPS3 CASSETTES

The DVIVPS1 (CIVPS3)-β-galactosidase fusions containing cassettes with a threonine or cysteine to substitute the serine at the carboxyl termini exhibit thermal-controllable cleavage at a specific peptide bond in the fusion proteins. The constructs described above (CIVPS3 cassettes inserted into the *lac*Z *Eco*RV site) yield fusion proteins after induction by IPTG. Cell extracts prepared from cells grown at 25°C were treated at elevated temperatures (42°C or 65°C) and analyzed by Western blots using antibody against β-galactosidase (Promega) or I*-Psp*I (the product of Deep Vent CIVPS3) (Figres 11 and 12). The IVPS1/Ser fusion protein can undergo protein splicing to generate a ligated protein and free IVPS endonuclease by incubation at elevated temperatures. While no ligation activity was observed, the fusion proteins with the CIVPS3/Thr or CIVPS3/Cys cassette cleave dominantly at the amino splice jnction at 42°C and both fuuion proteins exhibit increased cleavage activity at the carboxyl splice jnction at 65°C.

Preparation of cell extracts from the CIVPS3-*lac*Z fusion clones were performed as follows. All the fusion constructs originally constructed in different *E*. *coli* hosts were introduced into a *lac*Z-deletion *E*. *coli* strain ER1991 (New England Biolabs, Inc.), which did not synthesize β-galactosidase, by the standard transformation procedure as described in Example 8. A single colony from pDV7, pDVC302, pDVT332 or pDVS712 clone was inoculated in 1.5 ml LB medium supplemented with 100 µg/ml ampicillin, incubated at 30°C until OD₆₀₀ₙₘ reached about 0.5 and induced with 0.3 mM IPTG by adding 1.5 ml of 0.6 mM IPTG, 100 µg ampicillin/ml LB at 25°C for 5 hours. 3 ml of cells were spun down and resuspended in 0.5 ml of LB, sonicated for 1 min. at 4°C and spun at 6,000 rpm for 5 min. at 4°C. The supernatants were recovered and stored at -20°C.

The cell extracts were heat-treated at 42°C or 65°C after being quickly thawed at room temperature. The untreated control sample was prepared by mixing 48 µl of extract with 12 µl of 5 x sample buffer (0.31 Tris-HCI, pH 6.8/10% SDS/25% 2-mercaptoethanol/50% glycerol/0.005% Bromophenol ble), followed by boiling for 10 min. Aliquots of 48 µl were transferred into 1.5 ml microfuge tubes and incubated for 30, 60, 120, or 240 min. in a 42°C water bath, or 15, 30, 60 or 120 min. in a 65°C water bath. Each was mixed with 12 µl of 5 x sample buffer and boiled for 10 min.

The treated samples were analyzed by Western blots using antibodies raised against I-*Psp*I (NEB) (Figure 11 and 12) or "β-galactosidase (Promega) (Figure 12), 5 µl of each sample was loaded on 4/20% SDS polyacrylamide gels (ISS, Daichi, Tokyo, Japan) and electrophoresed at 100 volts for 4 hours. Western blots were carried out according to the procedure of Promega.

The results show that fusion protein precursors were the dominant species and barely trace amounts of I*-Psp*l endonuclease were present in cells after IPTG induction at 25°C from all for fusion constructs, indicating inefficient splicing and excision activity at low temperature. However, after shifting the pDVS712 (CIVPS3/Ser-β-galactosidase fusion) extract to higher temperatures, 42°C or 65°C, abundant CIVPS3 product, I-*Psp*I, (of about 60 KDa) accumulated (Figures 11 and 12). Excision of the IVPS domains was coupled with ligation of the N-domain and the C-domain of the interrupted β-galactosidase, producing a product of 116 KDa, identical in size to full-length β-galactosidase (Figure 12). Another major product (IVPS1-C-EPS) of about 130 KDa (corresponding to cleavage at the amino splice junction) was observed.

The fusion proteins of the other three variants (with CIVPS3, CIVPS3/Cys and CIVPS3/Thr cassettes) were more stable at low temperature. Very little I-*Psp*I or other products corresponding to cleavage at splice junctions were detected from the untreated extracts (Figure 11). In contrast to the CIVPS3/Ser fusion, no ligated proteins were observed from the heat-treated samples of these three fusion constructs (Figure 12). The pDV7 (ClVPS3-β-galactosidase fusion) sample produced only trace amounts of I-*Psp*I and products corresponding to cleavage at single splice junctions at 65°C, indicating poor excision at either splice junction (Figure 11, lanes 1-3). pDVC302, containing CIVPS3/Cys cassette, showed accumulation of moderate amounts of I*-Psp*l and CIVPS3-C-EP5 species at 42°C (Figure 11, lane 5). The yield in I*-Psp*l, C-EPS and a product (N-EPS-CIVPS3) of about 110 KDa, corresponding to cleavage at the carboxyl splice junction, was increased at 65°C while CIVPS-C-EPS species is reduced (Figure 11, lanes 4-6; Figure 12). The results indicate that the peptide bond cleavage at the carboxyl splice junction from the fusion protein and/or CIVPS-C-EPS product was enhanced. pDVT321 (with CIVPS3/Thr cassette), when treated at 42°C, showed very little I*-Psp*l or C-EPS but a dominant product, CIVPS3-C-EPS (Figure 11, lane 8; Figure 12). The data indicates efficient cleavage of the peptide bond at the amino splice junction but not at the carboxyl splice junction at 42°C. The accumulation of small amount of I*-Psp*l at 65°C indicated that cleavage at the carboxyl splice junction is enhanced (Figure 11, lane 9).

In summary, the data has demonstrated that by substitution of a single native reside, serine, at the carboxyl splice junction of the Deep Vent IVPS1 (CIVPS3), processing of the fusion proteins is altered and can be better controlled by temperature. The CIVPS3/Thr-β-galactosidase fusion protein (and CIVPS3/Cys fusion protein at a lesser extent) efficiently cleaved the specific peptide bond at the amino splice junction only at elevated temperatures.

### EXAMPLE 9

### CONSTRUCTION AND PURIFICATION OF MIP

### PURIFICATION OF CIVPS FUSIONS BY AFFINITY CHROMATOGRAPHY CLONING OF THE DEEP VENT IVPS1 INTO AN MBP FUSION PROTEIN

In one embodiment of the present invention a three-part fusion can be generated comprising a CIVPS; a segment which can be easily purified, e.g., a binding protein; and a protein or peptide of interest, i.e., a target protein. The order of these parts can be varied. The advantage of such a fusion is that it can be easily purified. Once the precursor protein is purified, the peptide of interest can be separated from the fusion by unidirectional protein cleavage induced by a modified CIVPS. In previous Examples, we have shown that if one of the CIVPS junctions is modified to reduce or prevent splicing or cleavage at that junction, then cleavage at the other junction will be favored over splicing (see, pages 13 and 14 and Example 8). This allows for separation of the peptide of interest away from the fusion.

This Example demonstrates that such a 3-part fusion composed of a binding protein, maltose binding protein (MBP), CIVPS3 and a paramyosin peptide can be easily purified on an amylose resin as an unspliced precursor. The precursor can then be induced to splice, in this case by thermal activation. In this Example, no attempt has been made to limit cleavage to one side of the CIVPS so as to interfere with splicing to generate only cleavage products without ligation.

### SYNTHESIS OF DEEP VENT IVPS1 INSERT (CIVPS3)

A CIVPS3 cassette was synthesized by PCR as described in previous Examples, with the following modifications. The PCR mixture contained Vent DNA polymerase buffer (NEB), 200M of each dNTP, 10pmoles of each primer, 40ng of plasmid DNA and 2 units of Vent DNA polymerase in 1001. Amplification was carried out using a Perken-Elmer thermal cycler at 94°C for 30 sec, 50°C for 30 sec and 72°C for 2 min for 20 cycles. Deep Vent IVPS1 was synthesized from pNEB #720.

The forward primer was, Primer 96-6, 5'-GGTACCCGTCGTGCTAGCATTTTACCGGAAGAATGGGTACCA-3'(SEQ ID NO:43), consisting of 26/27 bases at the 3' end which are identical to the 5' end of DV IVPS1, including 2 flanking *Kpn*I sites. The 3' *Kpn*I site includes a silent substitution which creates the restriction site without changing the amino acid residue. Deep Vent IVPS1 reverse primer, Primer 96-7, 5'-C CCGCTATTATGTGCATAGAGGGATCC-3' (SEQ ID NO:44) has a *Bam*HI site at the 3' end. 23/24 bases at the 3' end are homologous to the 3' end of DV IVPS1, with a single base substitution to create the *Bam*HI site. Primers 96-6 and 96-7 were used to synthesize the Deep Vent IVPS1 cassette (1.6kb).

The PCR sample was mixed 1:1 with chloroform and the top aqeous layer was loaded on a 1 % low melt agarose gel for electrophoresis. The 1.6 kb band was excised from the gel and incubated at 65°C. After the gel melted, 0.25ml TE buffer (10mM Tris-HCl/0.1mM EDTA, pH7.5) at 65°C was added and the sample was phenol-chloroform extracted (1:1 mixtre). The DNA was precipitated in 0.5M NaCI and 2 volumes isopropanol at -20°C for 30 min. The DNA was spun down, dried and resuspended in 60µl TE bffer.

### PREPARATION OF pPR1002, A pMAL-c2-PARAMYOSIN ΔSal PLASMID

pPR1002, a pMAL-c2-paramyosin ΔSal fusion plasmid, is a 7.2 kb vector that contains a *tac* promoter driven malE gene linked to an *Eco*RI-*Sal*I fragment of the *D. immitis* Paramyosin gene, referred to as the paramyosin ΔSal deletion (Steel, et al., *J*. *Immnology*, 145:3917-3923 (1990)). Two samples of 4 µg each of pPR1002 were linearized with 6 units of *Xmn*I (NEB) in 20 µl of 1X NEB bffer #2 containing 100 µg/ml BSA at 37°C for 2 hours. The reactions were loaded onto a 1 % low melting agarose gel. The 7.2 kb band was excised and purified from the gel as above, and resuspended in 40µl of TE buffer.

### CONSTRUCTION OF pMIP17

Ligation of pPR1002 and Deep Vent IVPS1 was carried out at 16°C for 16 hours in a 25µl volume with addition of 14.5µl distilled water, 2.5µl of 10X T4 DNA ligase buffer (NEB), 1µg/µg of cleaved pPR1002 DNA, 5µl of 0.2µg/µl Deep Vent IVPS1 prepared as described above and 800 units of T4 DNA ligase (NEB).

*E*. *coli* strain ER2252 was transformed on ice for 5 min. by mixing 100µl of competent ER2252 cells with 5µl of ligation sample in 100µl of a 1:2 mix of 0.1MCaCl₂ and 1XSSC (0.15M NaCI, 15mM NaCitrate), heating at 42°C for 3 min., chilling in ice for 5 min, adding 0.1ml LB media (10 grams/liter tryptone, 5 grams/liter yeast extract, 10 grams/liter NaCI, 1 gram/liter Dextrose, 1 gram/liter MgCl₂·6H₂O, pH7.2 at 25°C) and incubating for 30 min. at 30°C. 300 µl of transformed cells were pelleted and resuspended in 100µl supernatant and plated onto an LB amp plate. After incubation overnight at 30°C, about 160 colonies were observed.

PCR amplification was utilized to screen for colonies that carried recombinant plasmids. Individual colonies were picked into 100µl of distilled water in a 96 well microtitre dish, and boiled for 5 min to lyse the cells. The PCR mixture contained Vent DNA polymerase buffer (NEB), 200µM of each dNTP, 10pmoles of each primer (same as above), 2.5µl of cell lysate and 2 units of Vent Exo⁻ DNA polymerase in a 50µl reaction. Amplification was carried out by using a Perkin-Elmer thermal cycler at 94°C for 30 sec, 50°C for 30 sec and 72°C for 2 min for 30 cycles. 10µl of each reaction was run on a 1 % agarose gel. The positive clones had bands corresponding to IVPS1 (1.6kb) and one positive plasmid was designated pMIP17.

### EXPRESSION OF MIP: THE MBP-DEEP VENT IVPS1-PARAMYOSIN ΔSal FUSION

Positive clones containing pMIP17 were cultured in LB media supplemented with 100µg/ml ampicillin at 30°C until OD₆₀₀ₙₘ reached 0.5. To prepare a lysate from uninduced cells, 1.0ml of culture was pelleted and resuspended in 50µl Protein sample buffer (125mM Tris, 700mM β-mercaptoethanol, 2% SDS, 15% glycerol and 1mg/ml Bromophenol Blue). Samples from induced cultures were prepared as follows. After addition of IPTG to a final concentration of 1mM, the cultures were grown at 30°C for 20 additional hours. Cells from 0.5 ml culture at 5 hours and 20 hours after induction were pelleted and then resuspended in 100µl 5X protein sample buffer. The pre-induction and 5-hour samples were frozen at -20°C for 16 hours and the 20-hour sample was frozen at -70°C for 15 minutes. To improve precursor yield, cultures were induced at 12°C-20°C and amounts of precursor determined by Coomassie Blue stained gel. All the samples were boiled for 5 minutes and the protein products were analyzed by electrophoresis in SDS-PAGE followed by Coomassie Blue staining or Western blots using antibody raised against I*-Psp*l (NEB). The samples were electrophoresed on 4-20% SDS gels (ISS, Daichi, Tokyo, Japan) with prestained markers (BRL), transferred to nitrocellulose, probed with antisera (mouse anti-I-*Psp*l), and detected using alkaline phosphate-linked anti-mouse secondary antibody as described by the manufacturer (Promega). A predicted major band at about 132kDa was observed in both the Coomassie Blue stained gels and Western blots (data not shown).

### LARGE SCALE PURIFICATION OF THE MBP-DEEP VENT IVPS1 PARAMYOSIN ΔSal FUSION ON AMYLOSE AND MONOQ COLUMNS

Single colonies were used to inoculate 4x10ml LB media supplemented with 100µg/ml ampicillin and incubated at 30°C until OD₆₀₀ₙₘ reached 0.5. These cultures were used to inoculate 4x1 litre LB media supplemented with 100µg/ml ampicillin and incubated at 30°C until OD₆₀₀ₙₘ reached 0.5. The cultures were then transferred to 12°C and induced with 1mM IPTG overnight. The cells were pelleted and resuspended in column buffer (20mM NaPO₄ pH7.4, 200mM NaCI and 1mM EDTA), sonicated, spun down and the cleared culture lysate loaded over amylose resin (NEB Protein fusion and purification system). Fusion protein was eluted with maltose (as described by the manufacturer) and examined on an SDS-PAGE gel (Figure 13). The amylose resin elute was further purified by chromatography on FPLC MonoQ anion exchange resin (Pharmacia). The column was washed with 0.2 M NaCI, 10 mM Tris-HCI, pH8.5 and eluted with a linear gradient of NaCI from 0.2 to 1.0 M in 10 mM Tris-HCI, pH8.5. Protein eluted between 0.4-0.6M NaCI.

Six protein bands were identified by Western blot with antibodies to MBP, I-*Psp*l and paramyosin. Two bands of apparent molecular mass 180kDa and 132kDa reacted with all three antibodies. The full length precursor should be 132kDa. The higher molecular weight band is thought to be a splicing intermediate and similar high molecular weight species have been seen with all CIVPS constructs. The excised I*-Psp*l ran at 60kDa and was only recognized by the I-*Psp*I antibody, and the spliced product (MBP-Paramyosin ΔSal, 72kDa) was only recognized by sera reactive with the MBP and Paramyosin antibodies. A band of approximately 103 kDa reacted with only the MBP and I-*Psp*l antibodies and represents the product of a single cleavage at the C terminus of the IVPS. A band of approximately 89kDa reacted with only the I-*Psp*I and Paramyosin antisera and represents the product of a single cleavage at the N terminus of the IVPS (Figure 13).

### EXCISION AND LIGATION OF THE MBP-DEEP VENT IVPS1-PARAMYOSIN ΔSal FUSION

Amylose resin and MonoQ preparations containing several MIP-related polypeptides, including precursor (132kDa), slowly migrating species (180kDa apparent molecular mass), products of cleavage at a single splice junction (103kDa and 89kDa), and small amounts of spliced and excised products (72kDa and 60kDa) were heat-treated at 37°C for 2 hours in 20 mM sodium phosphate (pH6.0) and 0.5 M NaCI. The 132kDa precursor and 180kDa slowly migrating species decreased with time, while both the 72kDa spliced product and the 60kDa excised I-*Psp*l increased (Figure 13).

These results indicate that not only is it possible to purify 3-part CIVPS fusions, but that it is also possible to obtain single cleavage products. Further manipulation of the CIVPS junctions can favor cleavage at either splice junctions without ligation.

### EXAMPLE 10

### MODIFICATION OF CIVPS IN MIP FUSIONS

### CONSTRUCTION OF MIP WITH REPLACEABLE SPLICE JUNCTION CASSETTES

In this Example, an MIP fusion (see Example 9) with replaceable cassettes at both splice junctions and modification of the CIVPS by cassette substitution was constructed. We also show in two cases that modified CIVPSs are capable of cleavage at predominantly a single splice junction in a thermal inducible manner.

In Example 9, we described a three part fusion, MIP, that can be generated with the following properties: a CIVPS, a binding domain which can be easily purified (MBP) and a gene of interest (Paramyosin ΔSal). Splicing of the purified fusion protein yielded two major products, the ligated protein domains, MBP-paramyosin ΔSal, and the excised CIVPS (or I*-Psp*I). We reasoned that some modifications in the CIVPS may result in inhibition of the ligation reaction and enhancement of cleavage at one splice junction. This would result in separating the peptide of interest from the fusion protein by cleavage at a specific peptide bond catalyzed by a modified CIVPS. In Example 8, we have shown that cleavage at one splice junction can be enhanced by modification of CIVPS3 (substitution of the C-terminal Ser by Thr or Cys) and that these changes reduce or prevent splicing or cleavage at the other junction. In order to screen for modifications with favorable properties of controllable splicing or cleavage activity, it is necessary to introduce and analyze various mutations at the splice junctions. This could be accomplished by synthesis of the entire CIVPS cassette carrying each modification. However, this is likely to introduce extra mutations during PCR.

We have developed a strategy to facilitate the process by replacing only a short stretch of DNA around the splice junctions. In this Example, we describe how the original MIP fusion of Example 9 has been modified to contain two unique restriction sites flanking each splice junction. In a cassette replacement, following restriction digestion, the short stretch of DNA between the two unique restriction sites at one of the splice junctions can be replaced by another short DNA cassette. In this example, we modified the pMIP17 fusion described in Example 9 to contain two unique restriction sites at each junction: a *Xho*I site and a *Kpn*l site flanking the amino splice junction and a *Bam*HI site and a *Stu*l site flanking the carboxyl splice junction (see Figure 14).

The MIP fusion with splice junction cassettes is constructed in two steps. First, the *Bam*Hl and *Stu*l sites were introduced as follows. 4 µg of pMIP17 (Example 9) was digested in 1x *Eco*RI buffer (NEB) with 0.5 units of *Eco*RI (NEB) in 50 µl at 37°C for 10 min. After electrophoretic separation in an 1 % agarose gel, linearized pMIP17 plasmid DNA (8.8 Kb) was purified by using a Genecleanll kit (BIO101). The purified pMIP17 DNA was digested in 1x *Bam*Hl buffer (NEB) supplemented with 100 µg/ml BSA, 40 units of *Bam*HI (NEB) at 37°C for 3 hours and then extracted with phenol and chloroform. QNA was precipitated in 0.3 M NaAcetate (pH5.2) and 50% 2-propanol at -20°C for 2 hours. DNA was recovered by spinning for 10 min at 10,000 rpm in a microfuge, dried and resuspended in 20 µl sterile water.

Prior to ligation with the vector, two complementary oligomers, MIP301F (5'-GATCCCTCTATGCACATAATTCAGGCCTC-3' (SEQ ID NO:46)) and MIP302R (5'-AATTGAGGCCTGAATTATGTGCATAGAGG-3' (SEQ ID NO:47)) were allowed to anneal to form a double-stranded linker, MIP301F/MIP302R. 50 pmols of oligomers MIP301F and MIP302R were incubated in 1 x T4 DNA ligase buffer (NEB) at 68°C for 15 min and slowly cooled to 20°C-30°C. 1 µg of *Eco*RI-*Bam*HI-digested pMIP17 DNA was ligated at 16°C for 14 hours in 35 µl 1x T4 ligase buffer (NEB) with 80 units of T4 DNA ligase (NEB) and 25 pmols of the linker MIP301F/MIP302R.

The resulting construct was termed pMIP18. The upstream *Xho*I and *Kpn*I sites were introduced into pMIP18 as follows. 2 µg of pMIP18 was digested at 37°C for 4 hours in 100 µl of 1x Buffer 2 (NEB), 100 µg/ml BSA and 20 units of *Kpn*l (NEB). Following electrophoretic separation, linear pMIP18 DNA was purified by using the Genecleanll kit (BIO101). Prior to ligation with the vector, two complementary oligomers, MIP521F (5'-GCTCGAGGCTAGCATTTTACCGGAAGAATGGGTAC-3' (SEQ ID NO:48)) and MIP522R (5'-CCATTCTTCCGGTAAAATGCTAGCCTCGAGCGTAC-3' (SEQ ID NO:49)) were allowed to anneal to form a double-stranded linker, MIP521F/MIP522R. 50 pmols of oligomers MIP301F and MIP302R were incubated in 1x T4 DNA ligase buffer (NEB) at 75°C for 15 min and slowly cooled to 20°C-30°C. 0.2 µg of digested pMIP18 was ligated at room temperature for 3 hours in 35 µl of 1x T4 DNA ligase buffer (NEB), 80 units of T4 DNA ligase (NEB) and 25 pmols of the linker MIP521F/MIP522R. In each case, the ligated DNA samples were used to transform *E*. *coli* strain ER2252 (NEB). The final construct, pMIP21, contains two unique restriction sites at each splice junction. There is a *Xho*I site and a *Kpn*I site surrounding the N-terminal splice junction and a *Bam*HI site and a *Stu*l site surrounding the C-terminal splice junction (Figure 14).

Western blot analysis was performed to examine expression of modified MIP21 fusion protein and splicing activity. ER2252 containing pMIP21 was cultured at 30°C in LB medium supplemented by 100 µg/ml ampicillin until OD₆₀₀ₙₘ reached 0.5. The culture was then induced by 1 mM IPTG at 30°C for 3 hours. 4.5 ml of the culture was pelleted, resuspended in 0.5 ml LB medium and sonicated on ice. The cleared supernatant was electrophoresed on a 4/20% polyacrylamide gel at 100 volts for 4 hours. A Western blot was probed with anti-MBP sera. The results indicate that splicing activity from the modified MIP21 fusion was indistinguishable from that of MIP17.

### MODIFICATION OF MIP21 BY SPLICE JUNCTION CASSETTE REPLACEMENT

In the modified MIP fusion construct, pMIP21, the amino splice junction cassette includes 8 amino acid residues between the *Xho*I and *Kpn*I sites and the carboxyl splice junction cassette contains a sequence coding for 6 amino acid residues between the *Bam*HI and *Stu*I sites. Splice junctions can be changed by replacing either the N-terminal *Xho*I-*Kpn*I cassette or the C-terminal *Bam*HI-*Stu*I cassette. In the case of the C-terminal cassette replacement, pMIP21 is first digested with *Bam*HI and *Stu*l. Complementary primers containing desired mutations are substituted for the original *Bam*HI-*Stu*I cassette. In this Example, two different junction cassettes were substituted for the MIP21 *Bam*HI-*Stu*I cassette.

In the following cassette replacement examples, we substituted Ala₅₃₅ by Lys or His₅₃₆ by Leu.

Complementary oligomers MIP303F (5'-GATCCCTCTATAAGCATAATTCAGG-3' (SEQ ID NO:50) and MIP304R (5'-CCTGAATTATGCTTATAGAGG-3' (SEQ ID NO:51)) were used to substitute residue Ala₅₃₅ by Lys. Complementary oligomers MIP311F (5'-GATCCCTCTATGCACTGAATTCAGG-3' (SEQ ID NO:52)) and MIP312R (5'-CCTGAATTCAGTGCATAGAGG-3' (SEQ ID NO:53)) were used to substitute His₅₃₈ by Leu. These two pairs of complementary oligomers were treated as described above to form a double-stranded linker. Both linkers contain compatible termini to replace the carboxyl splice junction cassette following *Bam*HI-*Stu*I cleavage of pMIP21. 2 µg of pMIP21 DNA was digested with 40 units of *Bam*HI (NEB) in 1 x *Bam*HI buffer (NEB) supplemented with 100 µg/ml BSA at 37°C for 4 hours, extracted with chloroform and precipitated in 0.3 M NaAcetate (pH5.2) and 50% 2-propanol at -20°C for 2 hours. DNA was recovered by spinning for 10 min at 10,000 rpm in a microfuge, dried and resuspended in 88 µl sterile water. The *Bam*HI-digested pMIP21 DNA was then digested with 40 units of *Stu*I (NEB) in 100 µl 1x Buffer 2 (NEB) at 37°C for 3 hours, extracted with chloroform, precipitated in 0.3 M NaAcetate (pH5.2) and 50% 2-propanol at -20°C for overnight. pMIP21 DNA was recovered by spinning for 10 min at 10,000 rpm in a microfuge, dried and resuspended in 30 µl sterile water. 0.1 µg *Bam*HI-*Stu*I digested DNA was ligated at 23°C for 6 hours with 6 pmols of linker MIP303F/MIP304R or MIP311F/MIP312R in 10 ul of 1x T4 DNA ligase buffer (NEB) in the presence of 40 units of T4 DNA ligase (NEB). The ligated DNA was used to transform *E. coli* RR1. pMIP23 contains the Ala₅₃₅ to Lys substitution and pMIP28 contains the His₅₃₆ to Leu substitution. Expression of the modified MIP fusions, MIP23 and MIP28, was tested by western blot analysis with anti-MBP antibody as described above. The results indicated that splicing activity was blocked in both fusion constructs. However, each modification resulted in increased cleavage activity at only one of the splice junctions. The Ala₅₃₅ to Lys substitution in MIP23 drastically enhanced cleavage activity at the carboxyl splice junction and the His₅₃₆ to Leu substitution in MIP28 showed strong amino splice junction cleavage.

### PURIFICATION OF MODIFIED MIP FUSION PROTEINS AND THERMAL INDUCIBLE CLEAVAGE ACTIVITY

Expression of the fusion proteins was induced at low temperature and MIP fusion proteins were purified by amylose resin columns. RR1 harboring pMIP23 or pMIP28 were cultured in 1 liter of LB medium supplemented with 100 µg/ml ampicillin at 30°C until OD₆₀₀ₙₘ reached 0.5. After the cultures were cooled on ice to about 15°C, IPTG was added to a final concentration of 0.3 mM, and the cultures were grown at 12°C-14°C for 12 additional hours. Cells were pelleted, immediately frozen at -70°C and stored at -20°C. The pellets were separately sonicated in column buffer (10mM Tris pH8.5, 500mM NaCI) and spun down. The cleared lysate from each MIP fusion was loaded over amylose resin (NEB Protein fusion and purification system), washed and eluted with maltose (as described in Example 9).

A purified sample of MIP23 was dialyzed in 20mM NaPO₄ pH6.0)/500mM NaCI at 4°C. The sample was then incubated at 4°C, 37°C, 50°C, and 65°C for one hour and then electrophoresed on a 4/20% SDS-PAGE gel followed by Coomassie Blue staining (Figure 15a). The gel shows that with an increase in temperature MIP23 does not form the ligated product (MP) or the excised product (I), as the original construct does but instead accumulates the C-terminal cleavage products (Ml, 103 kD and P, 29 kD).

A purified MIP28 sample was dialyzed in 20mM NaPO₄ pH6.0)/500mM NaCI at 4°C for 1.5 hours. The sample was then incubated at 4°C, 42°C, 50°C, and 65°C for one hour and mixed with 1/5 volume of 5x Protein sample buffer (125mM Tris,700mM b-mercaptoethanol, 2% SDS,15% glycerol and 1mg/ml Bromophenol Blue). The protein products were analyzed by a 4/20% SDS-PAGE followed by Coomassie Blue staining (Figure 15). The data indicated that splicing activity was completely blocked under these conditions. Cleavage activity at the amino splice junction was increased corresponding to the increase in temperature, yielding more MBP (M, 43 kD) and CIVPS3-paramyosin ΔSal (IP, 89 kD) at 65°C.

These results show that the splice junction cassette replacement method can be utilized to modify the splice junctions in a fusion construct and such modifications may result in drastic effects on splicing and cleavage activity. Furthermore, this data gives examples of constructs where cleavage at only one splice junction is observed in the absence of ligation and total excision of the CIVPS.

### EXAMPLE 11

### CONSTRUCTION AND PURIFICATION OF MIC

### REPLACEMENT OF FOREIGN GENE IN CIVPS FUSIONS

A three-part fusion protein (MIP), composed of a binding domain for easy purification, a splicing domain (CIVPS3), and a target protein (paramyosin), was constructed as described in Example 9. This construct was purified and shown to be able to splice by thermal activation. To test the ability of this system to accept different target proteins, paramyosin in the MIP construct was replaced by the chitin binding domain (CBD) from the *Saccharomyces cerevisiae* chitinase gene [Kuranda and Robbins, *J*. *Biological Chem*., 266(29):19758-19767 (1991)]. The ability of this second protein fusion to splice and form both ligated and excised products shows that this fusion method can be employed with other foreign proteins. In addition, the chitin binding domain can be used as an alternate binding protein for protein purification.

### SYNTHESIS OF THE CHITIN BINDING DOMAIN (CBD)

A chitin binding domain was synthesized by PCR as described in the previous examples, with the following modifications. The PCR mixture contained Vent DNA polymerase buffer (NEB), 200 µM of each dNTP, 10pmoles of each primer, 20ng of plasmid DNA and 1 unit of Vent DNA polymerase in 100 µl. Amplification was carried out using a Perkin-Elmer thermal cycler at 95°C for 30 sec, 55°C for 30 sec, and 72°C for 30 sec for 20 cycles. The chitin binding domain was synthesized from pCT30, a plasmid containing the *Saccharomyces cerevisiae* chitinase gene [Kuranda and Robbins, *J*. *Biological Chem*., 266(29):19758-19767 (1991)].

The forward primer, primer 99-02, 5'-GTCAGGCCTCTCAGACAGTACAGCTCGTACAT-3' (SEQ ID NO:54) has a *Stu*l site (AGGCCT (SEQ ID NO:55)) at the 5' end. 22 bases at the 3' end of the primer are identical to the 5' end of the chitin binding domain of the chitinase gene. The reverse primer, primer 99-03, 5'-CCCCTGCAGTTAAAAGTAATTGCTTTCCAAATAAG-3' (SEQ ID NO:56) has a *Pst*I site (CTGCAG (SEQ ID NO:57)) at the 5' end. 26 bases at the 3' end of the primer are identical to the antisense strand at the 3' end of the chitin binding domain of the chitinase gene. Primers 99-02 and 99-03 were used to synthesize the chitin binding domain cassette (270bp).

The PCR sample was extracted with phenol-chloroform (1:1 mixture) and the DNA was precipitated in 0.5M NaCI and 2 volumes isopropanol at -20°C for 30 min. The DNA was spun down, dried and resuspended in 40µl TE buffer. (10mM Tris-HCl, 0.1mM EDTA, ph 7.5) A digest containing 20µl of the resuspended DNA, 21µl distilled water, 5µl 10X NEB Buffer #2, 40 units *Pst*I (NEB) and 20 units *Stu*l (NEB) was then carried out at 37°C for two hours in a 50µl volume. The reaction was loaded on a 1.8% low melt agarose gel for electrophoresis. The 0.25kb *Pst*I/*Stu*I digested product was excised from the gel and incubated at 65°C until the gel melted. 0.25 ml TE buffer at 65°C was added and the sample was phenol-chloroform extracted (1:1 mixture). The DNA was precipitated in 0.5M NaCI and 2 volumes isopropanol at -20°C for 30 min, spun down, dried and resuspended in 40µl TE buffer.

### PREPARATION OF PMIP21

A *Pst*I/*Stu*I double digest separates the paramyosin coding region from the remainder of the pMIP21, described in Example 10. Two samples of 5µg each of pMIP21 were digested with 60 units *Pst*I (NEB) and 30 units *Stu*l (NEB), 5µl of NEB buffer #2, and 34µl distilled water in a 50µl volume at 37°C for two hours. The reactions were loaded onto a 1 % low melting agarose gel. The 8.1kb band was excised and purified from the gel as above, and resuspended in 40µl TE buffer.

### CONSTRUCTION OF MBP-DEEP VENT IVPS1-CBD FUSION (MIC)

The chitin binding domain was substituted for paramyosin in MIP21 as follows to create MBP-Deep Vent IVPS1-CBD constructs (MIC). 1µl of 8.1 kb pMIP21 fragment, 10µl of chitin binding domain (both prepared as described above) were combined with 9.5µl distilled water, 2.5µl of 10X T4 DNA ligase buffer (NEB), and 800 units of T4 DNA ligase (NEB) and incubated at 16°C for 4 hours in a 25µl volume.

*E*. *coli* strain RR1 *tonA* (NEB) was transformed by (1) mixing 100µl of competent RR1 *tonA* cells with 5µl of ligation sample and 100µl of a 1:2 mix 0.1MCaCl₂ and 1XSSC(0.15M NaCl, 15mM NaCitrate) on ice for 5 min., (2) heating at 42°C for 3 min., (3) chilling in ice for 5 min and (4) plating onto an LB amp plate. After incubation overnight at 30°C, about 200 colonies were observed.

Alkaline lysis mini-prep DNA (Sambrook, *supra*) was utilized to screen for clones that carry recombinant plasmids with the chitin binding domain. When digested with *Pst*I and *Stu*l, the positive clones had a band corresponding to chitin binding domain and a band corresponding to the vector. The restriction enzyme digests were carried out by mixing 10µl miniprep DNA, 2.5µl NEB buffer #2, 8.5 µl distilled water, 40 units *Pst*I (NEB) and 20 units *Stu*l (NEB) in a 25 µl volume at 37°C for 2 hours.

### EXPRESSION OF THE MIC FUSIONS

To verify MIC constructs, small scale protein preparations were analyzed on Coomassie Blue stained gels and western blots. The positive clones were cultured in LB Media supplemented with 100µg/ml ampicillin at 30°C until OD₆₀₀ reached approximately 0.5. To prepare lysate from uninduced cells, 1.5ml of culture was pelleted and resuspended in 25µl 5X Protein sample buffer (125mM Tris, 700mM b-Mercaptoethanol, 2% SDS, 15% glycerol and 1 mg/ml Bromophenol Blue). Protein samples from induced cultures were prepared as follows. After cooling the cultures to 12°C, IPTG was added to a final concentration of 1mM and the cultures were grown at 12°C for 5 additional hours. After 2 hours of induction, a 1.5ml sample was taken and after 5 hours of induction a 3ml sample was taken. Samples were pelleted, resuspended in 50µl 5X protein sample buffer, frozen at -20°C for 16 hours, and then, thawed and boiled for 5 minutes. The protein products were analyzed by Coomassie Blue stained gels and Western blots using anti-MBP antibody (NEB). The samples were electrophoresed on 4-20%SDS gels (ISS, Daichi, Tokyo, Japan) with prestained markers (BRL), transferred to nitrocellulose, probed with anti-MBP antibody, and detected using alkaline phosphate-linked anti-rabbit secondary antibody as described by the manufacturer (Promega). A predicted major band at about 110kDa for the MIC fusion protein was observed in both the Coomassie Blue stained gels and Western blots.

### LARGE SCALE PURIFICATION OF MIC ON AMYLOSE AND MONOQ COLUMNS

Single colonies were used to inoculate 3x10ml LB media supplemented with 100µg/ml ampicillin and incubated at 30°C overnight. These cultures were used to inoculate 3x1 liter LB media supplemented with 100µg/ml ampicillin and incubated at 30°C until OD₈₀₀ reached 0.5. The cultures were then transferred to 12°C and induced with 1 mM IPTG overnight. The cells were pelleted and resuspended in column buffer (10mM Tris-HCI pH8.5, 500mM NaCI), sonicated, spun down and the cleared culture lysate loaded over amylose resin (NEB Protein fusion and purification system). Fusion protein was eluted with maltose (as described by the manufacturer) and examined on an SDS-PAGE gel. The amylose resin eluate was further purified by chromatography on FPLC MonoQ anion exchange resin (Pharmacia). The column was washed with 0.2M NaCl, 10mM Tris-HCl pH8.5 and eluted with a linear gradient of NaCI from 0.2 to 1.0M NaCI in 10mM Tris-HCl,pH8.5. Protein eluted between 0.4-0.6M NaCI. The MIC and MIP protein fusion products purified similarily on both the amylose resin and the MonoQ resin.

### EXCISION AND LIGATION OF THE MBP-DEEP VENT IVPS1-CBD FUSION

An amylose purified sample of MIC was dialyzed to 20mM NaPO₄ pH6.0, 500mM NaCI. The sample was then heat treated at 4°C, 37°C, 50°C, and 65°C for one hour and then examined on an SDS-PAGE gel. (Figure 16) The gel shows an abundance of MIC precursor, approximately 110kDa, in the 4°C sample which decreases after thermal induction. Along with the decrease in precursor, an accumulation of ligated product of approxiamtely 53kDa in size, MBP-CBD(MC), and excised product of approxiamtely 60kDa in size, Deep Vent IVPS1(I=I-*Psp*I), is observed with the increase in temperature. Also, the gel shows that bands of the same size as cleavage products, MBP-Deep Vent IVPS1(MI), approximately 103kDa, and Deep Vent IVPS1-CBD(IC), approximately 70kDa, are present.

This invention has been described in detail including the preferred embodiments thereof. However, it will be appreciated that those skilled in the art, upon consideration of this disclosure, may make modifications and improvements thereon without departing from the spirit and scope of the invention as set forth in the claims.

## Claims

1. A fusion protein **characterized in that** it comprises two parts: an intervening protein sequence (IVPS) and a target protein, wherein the IVPS is optionally modified and is positioned within or adjacent the target protein to form a splice junction, the splice junction comprising at the C-terminal side an amino acid residue having an -OH or -SH side chain, and causes the fusion protein to cleave or splice on changing a predetermined condition.

2. A fusion protein as claimed in claim 1, wherein the IVPS is fused at the amino or carboxy terminal end of the target protein.

3. A fusion protein as claimed in claim 1 wherein the IVPS is positioned within the target protein such that the target protein, which is an enzyme, is rendered substantially inactive.

4. A fusion protein as claimed in claim 3 wherein, upon splicing of the IVPS from within the target protein, the activity of the target protein is substantially restored.

5. A fusion protein as claimed in claim 1 wherein the IVPS encodes an endonuclease having homology to a homing endonuclease.

6. A fusion protein as claimed in claim 5 wherein the endonuclease function of the IVPS has been substantially inactivated.

7. A fusion protein as claimed in claim 1 wherein splicing comprises trans-splicing.

8. A fusion protein as claimed in claim 1 wherein the IVPS is selected from CIVP51, CIVPS2 and CIVPS3.

9. A fusion protein as claimed in claim 1 wherein the IVPS is inserted immediately before a serine, threonine or cysteine residue of the target protein.

10. A fusion protein as claimed in claim 1 wherein the IVPS contains a serine, threonine or cysteine residue at its amino end.

11. A fusion protein as claimed in claim 1 wherein the IVPS contains a serine, threonine or cysteine residue at its carboxy end.

12. A fusion protein as claimed in claim 1 wherein the optional modification involves at least one residue having an -OH or -SH side-chain.

13. A fusion protein as claimed in claim 12 wherein the modification is a post-translational or co-translational chemical derivatization of the side-chain.

14. A fusion protein as claimed in claim 1 wherein the predetermined condition changed is selected from temperature, pH, light, phosphorylation or glycosylation.

15. A fusion protein as claimed in claim 1 wherein it comprises a binding protein having affinity for a substrate.

16. A fusion protein as claimed in claim 15 wherein the substrate is an antibody against the IVPS.

17. A method of producing a fusion protein as claimed in claim 1 **characterized in that** it comprises:
(a) joining a DNA encoding an IVPS, which has been rendered controllable, with a DNA encoding a target protein to form a fusion DNA;
and
(b) expressing the said fusion DNA to produce the modified target protein.

18. A method for purification of a target protein **characterized in that** it comprises:
(a) forming a fusion protein as claimed in claim 1 comprising an IVPS, which has been rendered controllable, a target protein and a binding protein having affinity for a substrate;
(b) contacting the fusion protein with a substrate to which the binding protein binds;
(c) subjecting the substrate bound fusion protein to conditions under which cleavage of the IVPS occurs, thus separating the target protein from the binding protein;
and
(d) recovering the target protein.

19. A method for purification of a target protein **characterized in that** it comprises:
(a) forming a fusion protein as claimed in claim 1 comprising an IVPS, which has been rendered controllable, and a target protein;
(b) contacting the fusion protein with a substrate to which the IVPS binds;
(c) subjecting the substrate bound fusion protein to conditions under which cleavage of the IVPS occurs, thus separating the target protein from the IVPS;
and
(d) recovering the target protein.

20. A method as claimed in claim 19 wherein the substrate is an antibody against the IVPS.

21. A method of rendering an intervening protein sequence (IVPS) controllable **characterized in that** it comprises:
(a) selecting an IVPS;
(b) optionally modifying the IVPS and positioning the IVPS within or adjacent a target protein to form a fusion protein such that cleavage or splicing occurs as a result of a change in a predetermined condition;
and
(c) determining whether such control has been provided in the fusion protein.

## Patentansprüche

1. Fusionsprotein, **dadurch gekennzeichnet, dass** es zwei Teile umfasst:
eine Intervening-Protein-Sequenz (IVPS) und ein Targetprotein, wobei die IVPS wahlweise modifiziert und innerhalb oder benachbart dem Targetprotein angeordnet ist, zur Bildung einer Spleißstelle, wobei die Spleißstelle an der C-terminalen Seite einen Aminosäurerest umfasst, der eine -OH oder -SH Seitenkette bildet und die Spaltung oder das Spleißen des Fusionsproteins bei Veränderung eines vorher bestimmten Zustandes verursacht.

2. Fusionsprotein nach Anspruch 1, wobei die IVPS am amino- oder carboxyterminalen Ende des Targetproteins fusioniert ist.

3. Fusionsprotein nach Anspruch 1, wobei die IVPS innerhalb des Targetproteins derart positioniert ist, dass das Targetprotein, das ein Enzym ist, im wesentlichen inaktiv gehalten wird.

4. Fusionsprotein nach Anspruch 3, wobei die Aktivität des Zielproteins nach Spleißen der IVPS von innerhalb des Zielproteins im wesentlichen wiederhergestellt wird.

5. Fusionsprotein nach Anspruch 1, wobei die IVPS eine Endonuklease mit Homologie zu einer Homing-Endonuklease kodiert.

6. Fusionsprotein nach Anspruch 5, wobei die Endonukleasefunktion der IVPS im wesentlichen inaktiviert wurde.

7. Fusionsprotein nach Anspruch 1, wobei das Spleißen ein Trans-Spleißen umfasst.

8. Fusionsprotein nach Anspruch 1, wobei die IVPS aus CIVPS1, CIVPS2 und CIVPS3 ausgewählt ist.

9. Fusionsprotein nach Anspruch 1, wobei die IVPS unmittelbar vor einem Serin-, Threonin- oder Cysteinrest des Zielproteins eingefügt ist.

10. Fusionsprotein nach Anspruch 1, wobei die IVPS ein Serin-, Threonin- oder Cysteinrest an ihrem Amino-Ende enthält.

11. Fusionsprotein nach Anspruch 1, wobei die IVPS einen Serin-, Threonin- oder Cysteinrest an seinem Carboxy-Ende enthält.

12. Fusionsprotein nach Anspruch 1, wobei die wahlweise Modifikation zumindest einen Rest mit einer -OH oder -SH-Seitenkette einschließt.

13. Fusionsprotein nach Anspruch 12, wobei die Modifikation eine post-translationale oder co-translationale chemische Derivatisierung der Seitenkette ist.

14. Fusionsprotein nach Anspruch 1, wobei der vorherbestimmte Zustand, der geändert wird, aus Temperatur, pH, Licht, Phosphorylierung oder Glykosylierung ausgewählt ist.

15. Fusionsprotein nach Anspruch 1, das ein Bindungsprotein mit einer Affinität für ein Substrat umfasst.

16. Fusionsprotein nach Anspruch 15, wobei das Substrat ein Antikörper gegen die IVPS ist.

17. Verfahren zur Herstellung eines Fusionsproteins nach Anspruch 1, **dadurch gekennzeichnet, dass** es folgendes umfasst:
(a) Verbinden einer DNA, die eine IVPS kodiert, die kontrollierbar gemacht wurde, mit einer DNA, die ein Targetprotein kodiert, zur Bildung einer Fusions-DNA; und
(b) Exprimieren der Fusions-DNA zur Erzeugung des modifizierten Targetproteins.

18. Verfahren zur Aufreinigung eines Targetproteins, **dadurch gekennzeichnet, dass** es folgendes umfasst:
(a) Ausbilden eines Fusionsproteins nach Anspruch 1, das eine IVPS, die kontrollierbar gemacht wurde, ein Targetprotein und ein Bindungsprotein mit einer Affinität für ein Substrat umfasst;
(b) Inberührungbringen des Fusionsproteins mit einem Substrat, an das das Bindungsprotein bindet;
(c) Unterwerfen des substratgebundenen Fusionsproteins gegenüber Bedingungen, unter denen eine Spaltung der IVPS eintritt, wodurch das Targetprotein vom Bindungsprotein getrennt wird;
und
(d) Wiedergewinnen des Zielproteins.

19. Verfahren zur Aufreinigung eines Targetproteins, **dadurch gekennzeichnet, dass** es folgendes umfasst:
(a) Ausbilden eines Fusionsproteins nach Anspruch 1, das eine IVPS, die kontrollierbar gemacht wurde, und ein Targetprotein umfasst;
(b) Inberührungbringen des Fusionsproteins mit einem Substrat, das die IVPS bindet;
(c) Unterwerfen des substratgebundenen Fusionsproteins gegenüber Bedingungen, unter denen die Spaltung der IVPS eintritt, wodurch das Targetprotein von der IVPS abgetrennt wird;
und
(d) Wiedergewinnen des Zielproteins.

20. Verfahren nach Anspruch 19, wobei das Substrat ein Antikörper gegen die IVPS ist.

21. Verfahren, eine Intervening-Protein-Sequenz (IVPS) kontrollierbar zu machen, **dadurch gekennzeichnet, dass** es folgendes umfasst:
(a) Auswählen einer IVPS;
(b) Wahlweise modifizieren der IVPS und Positionieren der IVPS innerhalb oder angrenzend an ein Targetprotein zur Bildung eines Fusionsproteins, derart, dass eine Spaltung oder ein Spleißen als Folge einer Veränderung eines vorherbestimmten Zustandes eintritt;
und
(c) Bestimmen, ob eine solche Kontrolle im Fusionsprotein bereitgestellt wurde.

## Revendications

1. Protéine hybride **caractérisée en ce qu'**elle comprend deux parties : une intéine (IVPS, Intervening Protein Sequence) et une protéine cible, dans laquelle l'intéine est facultativement modifiée et est positionnée à l'intérieur ou à côté de la protéine cible pour former une jonction d'épissage, la jonction d'épissage comprenant, du côté C-terminal, un résidu d'acide aminé ayant une chaîne latérale -OH ou -SH, et qui fait que la protéine hybride est clivée ou épissée quand on change une condition prédéterminée.

2. Protéine hybride selon la revendication 1, dans laquelle l'intéine est fusionnée au niveau de la terminaison amino ou carboxy de la protéine cible.

3. Protéine hybride selon la revendication 1, dans laquelle l'intéine est positionnée à l'intérieur de la protéine cible, de telle manière que la protéine cible, qui est une enzyme, est rendue sensiblement inactive.

4. Protéine hybride selon la revendication 3, dans laquelle, lors de l'épissage de l'intéine à l'intérieur de la protéine cible, l'activité de la protéine cible est sensiblement restaurée.

5. Protéine hybride selon la revendication 1, dans laquelle l'intéine code pour une endonucléase ayant une homologie avec une endonucléase de "homing".

6. Protéine hybride selon la revendication 5, dans laquelle la fonction endonucléase de l'intéine a été sensiblement inactivée.

7. Protéine hybride selon la revendication 1, dans laquelle l'épissage comprend le trans-épissage.

8. Protéine hybride selon la revendication 1, dans laquelle l'intéine est choisie parmi le CIVPS1, le CIVPS2 et le CIVPS3.

9. Protéine hybride selon la revendication 1, dans laquelle l'intéine est insérée immédiatement avant un résidu sérine, thréonine ou cystéine de la protéine cible.

10. Protéine hybride selon la revendication 1, dans laquelle l'intéine contient un résidu sérine, thréonine ou cystéine au niveau de son extrémité amino.

11. Protéine hybride selon la revendication 1, dans laquelle l'intéine contient un résidu serine, thréonine ou cystéine au niveau de son extrémité carboxy.

12. Protéine hybride selon la revendication 1, dans laquelle la modification facultative implique au moins un résidu ayant une chaîne latérale -OH ou -SH.

13. Protéine hybride selon la revendication 12, dans laquelle la modification est une dérivation chimique post-traductionnelle ou co-traductionnelle de la chaîne latérale.

14. Protéine hybride selon la revendication 1, dans laquelle la condition prédéterminée changée est choisie parmi la température, le pH, la lumière, la phosphorylation ou la glycosylation.

15. Protéine hybride selon la revendication 1, qui comprend une protéine de liaison ayant une affinité pour un substrat.

16. Protéine hybride selon la revendication 15, dans laquelle le substrat est un anticorps contre l'intéine.

17. Procédé de production d'une protéine hybride selon la revendication 1, **caractérisé en ce qu'**il consiste à :
(a) relier un ADN codant pour une intéine, qui a été rendu contrôlable, à un ADN codant pour une protéine cible pour former un ADN de fusion ;
et
(b) exprimer ledit ADN de fusion pour produire la protéine cible modifiée.

18. Procédé de purification d'une protéine cible **caractérisé en ce qu'**il comprend les étapes consistant à :
(a) former une protéine hybride selon la revendication 1, comprenant une intéine, qui a été rendue contrôlable, une protéine cible et une protéine de liaison ayant une affinité pour un substrat ;
(b) mettre en contact la protéine hybride avec un substrat auquel la protéine de liaison est liée ;
(c) soumettre la protéine hybride liée au substrat aux conditions dans lesquelles le clivage de l'intéine se produit, ce qui permet ainsi de séparer la protéine cible de la protéine de liaison ;
et
(d) récupérer la protéine cible.

19. Procédé de purification d'une protéine cible **caractérisé en ce qu'**il consiste à :
(a) former une protéine hybride selon la revendication 1, comprenant une intéine, qui a été rendue contrôlable, et une protéine cible ;
(b) mettre en contact la protéine hybride avec un substrat auquel l'intéine est liée ;
(c) soumettre la protéine hybride liée au substrat aux conditions dans lesquelles le clivage de l'intéine se produit, ce qui permet ainsi de séparer la protéine cible de l'intéine ;
et
(d) récupérer la protéine cible.

20. Procédé selon la revendication 19, dans lequel le substrat est un anticorps contre l'intéine.

21. Procédé utilisé pour rendre une intéine (IVPS) contrôlable, **caractérisé en ce qu'**il comprend les étapes consistant à :
(a) choisir uen intéine ;
(b) facultativement modifier l'intéine et positionner l'intéine à l'intérieur ou à côté d'une protéine cible pour former une protéine hybride, de telle manière que le clivage ou l'épissage se produisent suite à un changement de la condition prédéterminée ;
et
(c) déterminer si un tel contrôle a été fourni dans la protéine hybride.
